(19) Europäisches Patentamt
European Patent Office
Office européen des brevets

(11) Publication number: **0 455 247 B1**

(12) **EUROPEAN PATENT SPECIFICATION**

(45) Date of publication of patent specification: **13.09.95**

(51) Int. Cl.[6]: **C07C 211/54**, C07C 217/92, G03G 5/06

(21) Application number: **91107132.2**

(22) Date of filing: **02.05.91**

(54) **m-Phenylenediamine compound and electrophotosensitive material using said compound.**

(30) Priority: **02.05.90 JP 116132/90**
**02.05.90 JP 116133/90**
**02.05.90 JP 116134/90**
**02.05.90 JP 116135/90**

(43) Date of publication of application:
**06.11.91 Bulletin 91/45**

(45) Publication of the grant of the patent:
**13.09.95 Bulletin 95/37**

(84) Designated Contracting States:
**DE FR GB IT**

(56) References cited:
**EP-A- 0 353 067**
**US-A- 4 992 350**

**PATENT ABSTRACTS OF JAPAN vol. 13, no. 395 (P-927)(3743), 4 September 1989; & JP-A-1142642**

**PATENT ABSTRACTS OF JAPAN vol. 14, no. 230 (P-1048)(4173), 16 May 1990; & JP - A - 2055361 (MITA IND CO LTD) 23.02.1990**

(73) Proprietor: **MITA INDUSTRIAL CO. LTD.**
**2-28, 1-chome, Tamatsukuri**
**Chuo-ku**
**Osaka-shi**
**Osaka 540 (JP)**

(72) Inventor: **Miyamoto, Eiichi**
**6-1-610, Nankohigashi 1-chome, Suminoe-ku**
**Osaka-shi, Osaka 559 (JP)**
Inventor: **Muto, Nariaki**
**Room No.301, Suiitopia-miyamoto, 1-1-10**
**Tashiden, Daito-shi, Osaka 574 (JP)**
Inventor: **Maeda, Tatsuo**
**2-3-17-310, Ogi, Higashinada-ku, Kobe-shi**
**Hyogo 658 (JP)**
Inventor: **Sumida, Keisuke**
**Room No.406, Nagao-ryo, 3-5-1**
**Fujisakahigashimachi**
**Hirakata-shi, Osaka 573-01 (JP)**
Inventor: **Kimura, Tadao**
**1-14-9, Aoyamadai, Tarumi-ku, Kobe-shi**
**Hyogo 655 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMISCHE BERICHTE vol. 103, no. 5, 1970, pages 1318-1333; P. WELZEL: "Die thermische Spaltung des Tetraphenylhydrazins. Ein Beitrag zum Problem der nicht katalysierten Benzidin-Umlagerung"**

**PATENT ABSTRACTS OF JAPAN vol. 13, no. 533 (P-967)(3881), 28 November 1989;& JP - A - 1219838 (FUJITSU LTD.) 01.09.1989**

(74) Representative: **Bohnenberger, Johannes, Dr. et al**
**Meissner, Bolte & Partner**
**Postfach 86 06 24**
**D-81633 München (DE)**

## Description

The present invention relates to m-phenylenediamine compounds suitable for an electric charge transferring material in an electrophotosensitive material, and also relates to an electrophotosensitive material using such compounds.

As an electrophotosensitive material in an image forming apparatus such as an electrophotographic copying apparatus, there is recently used an organic photosensitive material which is excellent in machinability and advantageous in production cost and which offers a great degree of freedom for design of performance.

For forming a copied image with the use of an electrophotosensitive material, the Carlson process is widely used. The Carlson process comprises the steps of uniformly charging a photosensitive material with electricity by corona discharge, exposing the charged photosensitive material to a document image, thereby to form an electrostatic latent image corresponding to the document image, developing the electrostatic latent image by a toner-containing developer, thereby to form a toner image, transferring the toner image to a medium such as paper, fixing the toner image transferred to the medium, and cleaning the photosensitive material to remove toner remaining thereon after the toner image has been transferred. To form an image of high quality in the Carlson process, it is required that the electrophotosensitive material be excellent in charging and photosensitive characteristics and presents a low residual potential after exposure to light.

Proper selection of electric charge transferring material is one of the important factors for achieving these requirements. As the electric charge transferring material, there have been proposed a variety of compounds such as polyvinyl carbazole compounds, oxadiazole compounds, pyrazoline compounds, hydrazone compounds and the like.

In these electric charge transferring materials, however, the drift mobility representing the electric charge transferring ability is relatively small. Further, since the dependency of the drift mobility upon the electric field intensity is great, the movement of electric charge in a low electric field is small. This makes it difficult that the residual potential disappears. Further, such materials are disadvantageously apt to be deteriorated due to irradiation of ultraviolet rays or the like.

In view of the problems above-mentioned, there have been also proposed a variety of N,N,N',N'-tetraphenyl-1,3-phenylenediamines as examples of a m-phenylenediamine compound of which dependency of the drift mobility upon the electric field intensity is small and which has a good compatibility with respect to resins (Japanese Unexamined Patent Publication Nos. 142659/1989, 36270/1990, 36269/1990 and 297559/1990). Such a m-phenylenediamine compound presents good light-exposure properties with respect to ultraviolet rays and the like. When actually used in an electrophotographic copying apparatus, this compound presents stable characteristics. However, if this compound is exposed to light for a long period of time or at a high temperature in case of trouble of the copying apparatus, this compound is disadvantageously damaged in an irrecoverable extent.

The publication Patent Abstracts of Japan, Vol. 13, No. 395 (P-927) [3743] September 4, 1989, discloses an m-phenylenediamene compound for use as a charge transfer material. The substitution groups $R^1$ to $R^5$ can be an alkyl group, alkoxy group or a halogen atom.

The published European Patent application EP-A 0 353 067 discloses a photosensitive material containing an m-phenylenediamene compound. The group R on the central benzine ring is hydrogen, alkyl, alkoxy or halogen. Four groups are substituted at the meta positions of the nitrogen atoms, each of which is a hydrogen atom, and alkyl group, an alkoxy group or a halogen atom, with the proviso that at least two of the four groups is an alkyl group, alkoxy group or a halogen atom.

The publication Patent Abstracts of Japan, Vol. 14, No. 230, (P1048) [4173] May 16, 1990, discloses a photosensitive material made by combining a specific m-phenylenediamene compound and a specific pyrolopyrrole compound. The substitution groups $R^1$ to $R^5$ of the m-phenylenediamene compound can be an alkyl group or an alkoxy group. The publication Patent Abstracts of Japan, Vol. 13, No. 533 (P967) [3881], November 28, 1989, discloses a specific triaminobenzine derivative as an electric charge transfer material in a photoconductive layer. Each nitrogen position has two substitution groups, each being a lower alkyl, lower alkoxy, optionally substituted aryl groups. The published European Patent application EP-A 0 390 195 discloses an m-phenylenediamene compound for use in a photosensitive material. Two of the phenyl rings have substitutions introduced in the para position, while two phenyl rings have substitutions in the meta positions. The substitution groups are defined such that if one of the para or meta substitute groups is a hydrogen atom, then the other para or meta group respectively should not be a hydrogen atom.

It is a main object of the present invention to provide m-phenylenediamine compounds excellent in light-exposure properties and photostability, and also to provide an electrophotosensitive material using such a compound.

According to the present invention, m-phenylenediamine compounds are provided as defined in claims 1, 2 and 3. An electrophotosensitive material is also provided as defined in claim 5.

The decrease in characteristics of a photosensitive material due to light exposure is generally caused by the formation, in the photosensitive material, of impurities which constitute a trap for the electric charge transferring material. In the m-phenylenediamine compound, a ring-closure reaction made between the center benzene ring and other phenyl groups is considered to be such a photo-deterioration reaction. It is believed that this ring-closure reaction is apt to take place because the electron density of molecules in the m-phenylenediamine compound is biased to the center benzene ring. In particular, the fourth and sixth positions of the center benzene ring are high in electron density, so that the molecular structure is liable to be attacked by an oxide such as oxygen. Accordingly, the inventors of the present invention have considered that, when the fourth and/or sixth positions of the center benzene ring are substituted and protected by substituting groups, the reactivity may be restrained, thereby to improve the compound in stability. After having conducted a variety of tests, the inventors have found the novel fact that, when the fourth and/or sixth positions of the center benzene ring are substituted by specific substituting groups, the photosensitive material can be effectively improved in photostability without injury to the electric charge transferring characteristics such as drift mobility and the like.

Accordingly, one of the m-phenylenediamine compounds of the present invention is represented by the following general formula (I):

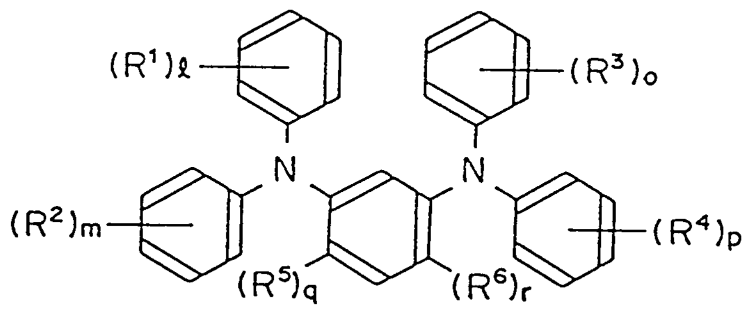

(I)

wherein $R^1$, $R^2$, $R^3$ and $R^4$ are the same or different from one another, and each is an alkyl group, an alkoxyl group, a halogen atom, an amino group or an N-substituting amino group; l, m, o and p are the same as or different from one another and each is an integer from 0 to 5; $R^5$ and $R^6$ are the same as or different from each other and each is an amino group, an N-substituting amino group, an alkenyl group or an aryl group; each of q and r is 0 or 1, but q and r should not be 0 simultaneously.

In the m-phenylenediamine compound (I), at least one of the fourth and sixth positions of the center benzene ring is protected by a substituting group (an amino group, an N-substituting amino group, an alkenyl group or an aryl group), and is therefore hardly attacked by an oxide. This remarkably restrains a photo-deterioration reaction to improve the compound in photostability.

Further, it is also possible to restrain the reactivity of the compound to improve the photostability by substituting and protecting, by a specific substituting group, the ortho-position of the phenyl group added to the nitrogen atom of the center benzene ring having a high electron density, as mentioned earlier. This results in effective improvement in the photostability of the photosensitive material without injury to the electric charge transferring characteristics such as drift mobility, likewise the case above-mentioned.

Accordingly, another m-phenylenediamine compound of the present invention is represented by the following general formula (II):

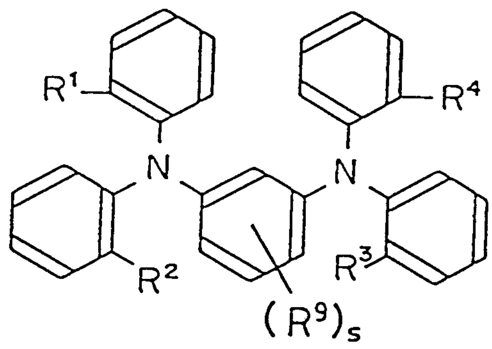

(II)

wherein $R^1$, $R^2$, $R^3$ and $R^4$ are as defined above; $R^9$ is an alkyl group, an alkoxyl group, a halogen atom, an amino group, an N-substituting amino group, an alkenyl group or an aryl group; s is an integer from 0 to 4.

In the m-phenylenediamine compound (II), the ortho-position of the phenyl group added to the nitrogen atom of the center benzene ring is substituted by a specific substituting group. Accordingly, the molecular electron density is averaged, causing the compound to be hardly attacked by an oxide. This restrains the ring-closure reaction to improve the photostability.

The fifth position of the center benzene ring having a high electron density presents a molecular structure which is liable, in view of steric configuration, to be attacked by an oxide such as oxygen at the time of light excitation. It is therefore considered that an electron is pulled out from this fifth position, thus generating the ring-closure reaction.

It is possible to substitute and protect this fifth position by a specific substituting group, thereby to restrain the reactibity to improve the stability. This results in effective improvement in the photostability of the photosensitive material without injury to the electric charge transferring characteristics such as drift mobility and the like.

Accordingly, a further m-phenylenediamine compound of the present invention is represented by the following general formula (III):

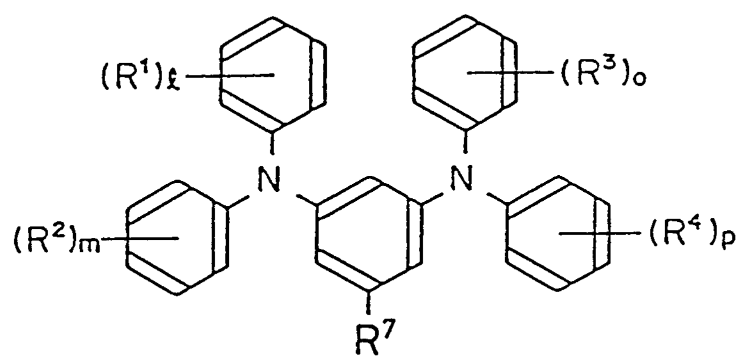

(III)

wherein $R^1$, $R^2$, $R^3$ and $R^4$ are as defined above; l, m, o and p are the same as or different from one another and each is an integer from 0 to 5; $R^7$ is an amino group, an alkenyl group, an aryl group or an electron attractive group.

In the m-phenylenediamine compound (III), the fifth position of the center benzene ring is protected by a specific substituting group $R^7$. Accordingly, this fifth position becomes hardly liable to attack by an oxide or the like. This remarkably restrains the photo-deterioration reaction to improve the compound in photostability. In particular, when the fifth position of the center benzene ring is substituted by the electron attractive group, the electron density distribution biased to the center benzene ring is made uniform, thereby to improve the photostability more than the case where this position is substituted by other substituting group than the electron attractive group. This is also presumed from variations of the electron density distribution in the basis state obtained by a molecular orbital calculation.

The photosensitive material containing any of the m-phenylenediamine compounds (I), (II) and (III) is less damaged by light-exposure for a long period of time or at a high temperature, than a conventional photosensitive material, and is therefore excellent in photostability.

Examples of the alkyl group include methyl, ethyl, propyl, isopropyl, butyl, isobutyl, tert-butyl, pentyl and hexyl groups, each having 1 to 6 straight-chain or branched carbon atoms.

Examples of the alkoxyl group include a lower alkoxyl group having 1 to 6 carbon atoms in its alkyl portion, such as methoxy, ethoxy, propoxy, isopropoxy, pentyloxy and hexyloxy groups.

Examples of the halogen atom include chlorine, bromine, iodine and fluorine.

Examples of the N-substituting amino group include an alkyl substituting amino group in which one or two alkyl groups having 1 to 6 carbon atoms as above-mentioned have been substituted.

Examples of the alkenyl group include vinyl, allyl, butenyl, methyl allyl, pentenyl and hexenyl group, each having straight-chain or branched 2 to 6 carbon atoms.

Examples of the aryl group include phenyl, tolyl, xylyl, biphenyl, naphthyl, antolyl and phenantolyl groups.

Examples of the electron attractive group include a halogen atom, and nitro, sulfo, cyano, $-COR^8$ - (wherein $R^8$ is a hydrogen atom, an alkyl group or an amino group), carboxyl and esterified carboxyl groups.

As specific examples of the m-phenylenediamine compound of the general formula (I), the following compounds are mentioned.

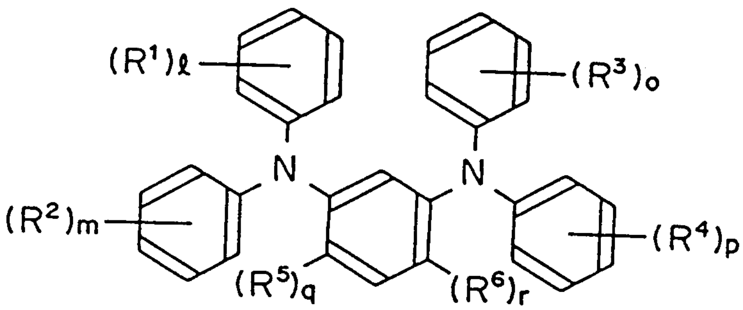

〔I〕

| $R^1$ | $R^2$ | $R^3$ | $R^4$ | $R^5$ | $R^6$ |
|---|---|---|---|---|---|
| $OC_2H_5$ | $CH_3$ | $CH_3$ | $OC_2H_5$ | $CH_3$ | $CH_3$ |
| $C_2H_5$ | $C_2H_5$ | $C_2H_5$ | $C_2H_5$ | $CH_2CH=CH_2$ | $CH_2CH=CH_2$ |
| $C_3H_7$ | $C_3H_7$ | $C_3H_7$ | $C_3H_7$ | $NH_2$ | $NH_2$ |
| $C(CH_3)_3$ | $C(CH_3)_3$ | $C(CH_3)_3$ | $C(CH_3)_3$ | $NHCH_3$ | $NHCH_3$ |
| $OCH_3$ | $OCH_3$ | $OCH_3$ | $OCH_3$ | $OCH_3$ | $OCH_3$ |
| $OC_2H_5$ | $OC_2H_5$ | $OC_2H_5$ | $OC_2H_5$ | $CH_3$ | $CH_3$ |
| H | $CH_3$ | $CH_3$ | H | $NH_2$ | $NH_2$ |
| $C_2H_5$ | $CH_3$ | $CH_3$ | $C_2H_5$ | $CH_2CH=CH_2$ | $CH_2CH=CH_2$ |
| $C(CH_3)_3$ | $CH_3$ | $CH_3$ | $C(CH_3)_3$ | $NHCH_3$ | $NHCH_3$ |
| $OCH_3$ | $CH_3$ | $CH_3$ | $OCH_3$ | $OCH_3$ | $OCH_3$ |

| $R^1$ | $R^2$ | $R^3$ | $R^4$ | $R^5$ | $R^6$ |
|---|---|---|---|---|---|
| H | H | H | H | $NH_2$ | $NH_2$ |
| $CH_3$ | $CH_3$ | $CH_3$ | $CH_3$ | $C_6H_5$ | $C_6H_5$ |
| $CH_3$ | $CH_3$ | $CH_3$ | $CH_3$ | $NH_2$ | $NH_2$ |
| $CH_3$ | $CH_3$ | $CH_3$ | $CH_3$ | $NH_2$ | $NH_2$ |
| $CH_3$ | $CH_3$ | $CH_3$ | $CH_3$ | $NHCH_3$ | $NHCH_3$ |
| $CH_3$ | $CH_3$ | $CH_3$ | $CH_3$ | $OCH_3$ | $OCH_3$ |
| $CH_3$ | $CH_3$ | $CH_3$ | $CH_3$ | $CH_3$ | $CH_3$ |
| $CH_3$ | H | H | $CH_3$ | $C_6H_5$ | $C_6H_5$ |
| $CH_3$ | $C_2H_5$ | $C_2H_5$ | $CH_3$ | $C_6H_5$ | $C_6H_5$ |
| $CH_3$ | $C_3H_7$ | $C_3H_7$ | $CH_3$ | $C_6H_5$ | $C_6H_5$ |
| $CH_3$ | $C(CH_3)_3$ | $C(CH_3)_3$ | $CH_3$ | $C_6H_5$ | $C_6H_5$ |
| $CH_3$ | $OCH_3$ | $OCH_3$ | $CH_3$ | $C_6H_5$ | $C_6H_5$ |
| $CH_3$ | $OC_2H_5$ | $OC_2H_5$ | $CH_3$ | $C_6H_5$ | $C_6H_5$ |
| H | $CH_3$ | $CH_3$ | H | $C_6H_5$ | $C_6H_5$ |
| $C_2H_5$ | $CH_3$ | $CH_3$ | $C_2H_5$ | $C_6H_5$ | $C_6H_5$ |
| $C_3H_7$ | $CH_3$ | $CH_3$ | $C_3H_7$ | $CH_3C_6H_4$ | $CH_3C_6H_4$ |
| $C(CH_3)_3$ | $CH_3$ | $CH_3$ | $C(CH_3)_3$ | $CH_3C_6H_4$ | $CH_3C_6H_4$ |
| $OCH_3$ | $CH_3$ | $CH_3$ | $OCH_3$ | $CH_3C_6H_4$ | $CH_3C_6H_4$ |

| $R^1$ | $R^2$ | $R^3$ | $R^4$ | $R^5$ | $R^6$ |
|---|---|---|---|---|---|
| $OC_2H_5$ | $CH_3$ | $CH_3$ | $OC_2H_5$ | $C_6H_5$ | $CH_2CH=CH_2$ |
| $CH_3$ | H | H | $CH_3$ | $CH_2CH=CH_2$ | $C_6H_5$ |
| $CH_3$ | $C_2H_5$ | $C_2H_5$ | $CH_3$ | $CH_2CH=CH_2$ | $CH_2CH=CH_2$ |
| $CH_3$ | $C_3H_7$ | $C_3H_7$ | $CH_3$ | $CH_2CH=CH_2$ | $CH_2CH=CH_2$ |
| $CH_3$ | $C(CH_3)_3$ | $C(CH_3)_3$ | $CH_3$ | $CH_2CH=CH_2$ | $CH_2CH=CH_2$ |
| $CH_3$ | $OCH_3$ | $OCH_3$ | $CH_3$ | $CH_2CH=CH_2$ | $CH_2CH=CH_2$ |
| $CH_3$ | $OC_2H_5$ | $OC_2H_5$ | $CH_3$ | $CH_2CH=CH_2$ | $NH_2$ |
| H | $CH_3$ | $CH_3$ | H | $CH_2CH=CH_2$ | $NH_2$ |
| $C_2H_5$ | $CH_3$ | $CH_3$ | $C_2H_5$ | $CH_2CH=CH_2$ | $NH_2$ |
| $C_3H_7$ | $CH_3$ | $CH_3$ | $C_3H_7$ | $CH_2CH=CH_2$ | $NH_2$ |
| $C(CH_3)_3$ | $CH_3$ | $CH_3$ | $C(CH_3)_3$ | $CH_2CH=CH_2$ | $NH_2$ |
| $OCH_3$ | $CH_3$ | $CH_3$ | $OCH_3$ | $CH_2CH=CH_2$ | $NH_2$ |
| $OC_2H_5$ | $CH_3$ | $CH_3$ | $OC_2H_5$ | $CH_2CH=CH_2$ | $NH_2$ |
| $CH_3$ | H | H | $CH_3$ | $NH_2$ | $CH_2CH=CH_2$ |
| $CH_3$ | $C_2H_5$ | $C_2H_5$ | $CH_3$ | $NH_2$ | $CH_2CH=CH_2$ |
| $CH_3$ | $C_3H_7$ | $C_3H_7$ | $CH_3$ | $NH_2$ | $CH_2CH=CH_2$ |
| $CH_3$ | $C(CH_3)_3$ | $C(CH_3)_3$ | $CH_3$ | $NH_2$ | $CH_2CH=CH_2$ |
| $CH_3$ | $OCH_3$ | $OCH_3$ | $CH_3$ | $NH_2$ | $CH_2CH=CH_2$ |

| $R^1$ | $R^2$ | $R^3$ | $R^4$ | $R^5$ | $R^6$ |
|---|---|---|---|---|---|
| $CH_3$ | $OC_2H_5$ | $OC_2H_5$ | $CH_3$ | $NH_2$ | $NHCH_3$ |
| H | $CH_3$ | $CH_3$ | H | $NH_2$ | $NHCH_3$ |
| $C_2H_5$ | $CH_3$ | $CH_3$ | $C_2H_5$ | $NH_2$ | $NHCH_3$ |
| $C_3H_7$ | $CH_3$ | $CH_3$ | $C_3H_7$ | $NH_2$ | $NHCH_3$ |
| $C(CH_3)_3$ | $CH_3$ | $CH_3$ | $C(CH_3)_3$ | $NH_2$ | $NHCH_3$ |
| $OCH_3$ | $CH_3$ | $CH_3$ | $OCH_3$ | $NH_2$ | $NHCH_3$ |
| $OC_2H_5$ | $CH_3$ | $CH_3$ | $OC_2H_5$ | $NH_2$ | $NHCH_3$ |
| $CH_3$ | H | H | $CH_3$ | $NHCH_3$ | $NH_2$ |
| $CH_3$ | $C_2H_5$ | $C_2H_5$ | $CH_3$ | $NHCH_3$ | $NH_2$ |
| $CH_3$ | $C(CH_3)_3$ | $C(CH_3)_3$ | $CH_3$ | $NHCH_3$ | $NH_2$ |
| $CH_3$ | $OCH_3$ | $OCH_3$ | $CH_3$ | $NHCH_3$ | $NH_2$ |
| $CH_3$ | $OC_2H_5$ | $OC_2H_5$ | $CH_3$ | $NHCH_3$ | $NH_2$ |
| H | $CH_3$ | $CH_3$ | H | $NHCH_3$ | $NH_2$ |
| $C_2H_5$ | $CH_3$ | $CH_3$ | $C_2H_5$ | $NHCH_3$ | H |
| $C(CH_3)_3$ | $CH_3$ | $CH_3$ | $C(CH_3)_3$ | $NHCH_3$ | H |
| $OCH_3$ | $CH_3$ | $CH_3$ | $OCH_3$ | $NHCH_3$ | H |
| $OC_2H_5$ | $CH_3$ | $CH_3$ | $OC_2H_5$ | $NHCH_3$ | H |
| $CH_3$ | H | H | $CH_3$ | $OCH_3$ | H |

| $R^1$ | $R^2$ | $R^3$ | $R^4$ | $R^5$ | $R^6$ |
|---|---|---|---|---|---|
| $CH_3$ | $C_2H_5$ | $C_2H_5$ | $CH_3$ | $OCH_3$ | H |
| $CH_3$ | $C(CH_3)_3$ | $C(CH_3)_3$ | $CH_3$ | $OCH_3$ | H |
| $CH_3$ | $OCH_3$ | $OCH_3$ | $CH_3$ | $OCH_3$ | H |
| $CH_3$ | $OC_2H_5$ | $OC_2H_5$ | $CH_3$ | $OCH_3$ | H |
| H | $CH_3$ | $CH_3$ | H | $OCH_3$ | $CH_3$ |
| $C_2H_5$ | $CH_3$ | $CH_3$ | $C_2H_5$ | $OCH_3$ | $CH_3$ |
| $C(CH_3)_3$ | $CH_3$ | $CH_3$ | $C(CH_3)_3$ | $OCH_3$ | $CH_3$ |
| $OCH_3$ | $CH_3$ | $CH_3$ | $OCH_3$ | $OCH_3$ | $CH_3$ |
| $OC_2H_5$ | $CH_3$ | $CH_3$ | $OC_2H_5$ | $OCH_3$ | $CH_3$ |
| $CH_3$ | H | H | $CH_3$ | $CH_3$ | $CH_3$ |
| $CH_3$ | $C_2H_5$ | $C_2H_5$ | $CH_3$ | $CH_3$ | $NH_2$ |
| $CH_3$ | $C(CH_3)_3$ | $C(CH_3)_3$ | $CH_3$ | $CH_3$ | $NH_2$ |
| $CH_3$ | $OCH_3$ | $OCH_3$ | $CH_3$ | $CH_3$ | $NH_2$ |
| $CH_3$ | $OC_2H_5$ | $OC_2H_5$ | $CH_3$ | $CH_3$ | $NH_2$ |

| $R^1$ | $R^2$ | $R^3$ | $R^4$ | $R^5$ | $R^6$ |
|---|---|---|---|---|---|
| Cl | Cl | Cl | Cl | Cl | Cl |
| $NH_2$ | $NH_2$ | $NH_2$ | $NH_2$ | $NH_2$ | $NH_2$ |
| $NHCH_3$ | $NHCH_3$ | $NHCH_3$ | $NHCH_3$ | $NHCH_3$ | $NHCH_3$ |
| $NH_2$ | Cl | $NH_2$ | Cl | $CH_3$ | $CH_3$ |
| $NH_2$ | Cl | $NH_2$ | Cl | $CH_2CH=CH_2$ | $CH_2CH=CH_2$ |
| $NH_2$ | Cl | $NH_2$ | Cl | $NH_2$ | $NH_2$ |
| $NH_2$ | Cl | $NH_2$ | Cl | $NHCH_3$ | $NHCH_3$ |
| $NH_2$ | Cl | $NH_2$ | Cl | $OCH_3$ | $OCH_3$ |
| $NH_2$ | Cl | $NH_2$ | Cl | $C_6H_5$ | $C_6H_5$ |
| $NH_2$ | Cl | $NH_2$ | Cl | $CH_3C_6H_4$ | $CH_3C_6H_4$ |
| $NH_2$ | $NHCH_3$ | $NH_2$ | $NHCH_3$ | $CH_3$ | $CH_3$ |
| $NH_2$ | $NHCH_3$ | $NH_2$ | $NHCH_3$ | $CH_2CH=CH_2$ | $CH_2CH=CH_2$ |
| $NH_2$ | $NHCH_3$ | $NH_2$ | $NHCH_3$ | $NH_2$ | $NH_2$ |
| $NH_2$ | $NHCH_3$ | $NH_2$ | $NHCH_3$ | $NHCH_3$ | $NHCH_3$ |
| $NH_2$ | $NHCH_3$ | $NH_2$ | $NHCH_3$ | $OCH_3$ | $OCH_3$ |
| $NH_2$ | $NHCH_3$ | $NH_2$ | $NHCH_3$ | $C_6H_5$ | $C_6H_5$ |
| Br | $NHCH_3$ | Br | $NHCH_3$ | $CH_3$ | $CH_3$ |
| Br | $NHCH_3$ | Br | $NHCH_3$ | $CH_2CH=CH_2$ | $CH_2CH=CH_2$ |

| $R^1$ | $R^2$ | $R^3$ | $R^4$ | $R^5$ | $R^6$ |
|---|---|---|---|---|---|
| Br | $NHCH_3$ | Br | $NHCH_3$ | $NH_2$ | $NH_2$ |
| Br | $NHCH_3$ | Br | $NHCH_3$ | $NHCH_3$ | $NHCH_3$ |
| Br | $NHCH_3$ | Br | $NHCH_3$ | $OCH_3$ | $OCH_3$ |
| Br | $NHCH_3$ | Br | $NHCH_3$ | $C_6H_5$ | $C_6H_5$ |
| Br | $NHCH_3$ | Br | $NHCH_3$ | $CH_3C_6H_4$ | $CH_3C_6H_4$ |
| Br | $NHCH_3$ | Br | $NHCH_3$ | $C_2H_5$ | $C_2H_5$ |
| $NH_2$ | $CH_3$ | $NH_2$ | $CH_3$ | $CH_3$ | $CH_3$ |
| $NH_2$ | $CH_3$ | $NH_2$ | $CH_3$ | $CH_2CH=CH_2$ | $CH_2CH=CH_2$ |
| $NH_2$ | $CH_3$ | $NH_2$ | $CH_3$ | $NH_2$ | $NH_2$ |
| $NH_2$ | $CH_3$ | $NH_2$ | $CH_3$ | $NHCH_3$ | $NHCH_3$ |
| $NH_2$ | $CH_3$ | $NH_2$ | $CH_3$ | $OCH_3$ | $OCH_3$ |
| $NH_2$ | $CH_3$ | $NH_2$ | $CH_3$ | $C_6H_5$ | $C_6H_5$ |
| $NH_2$ | $CH_3$ | $NH_2$ | $CH_3$ | $CH_3C_6H_4$ | $CH_3C_6H_4$ |
| $NH_2$ | $CH_3$ | $NH_2$ | $CH_3$ | $C_2H_5$ | $C_2H_5$ |
| Cl | $NH_2$ | Cl | $NH_2$ | $CH_3$ | $CH_3$ |
| Cl | $NH_2$ | Cl | $NH_2$ | $CH_2CH=CH_2$ | $CH_2CH=CH_2$ |
| Cl | $NH_2$ | Cl | $NH_2$ | $NH_2$ | $NH_2$ |
| Cl | $NH_2$ | Cl | $NH_2$ | $NHCH_3$ | $NHCH_3$ |

| $R^1$ | $R^2$ | $R^3$ | $R^4$ | $R^5$ | $R^6$ |
|---|---|---|---|---|---|
| $OC_2H_5$ | $CH_3$ | $CH_3$ | $OC_2H_5$ | H | $NH_2$ |
| $OC_2H_5$ | $OC_2H_5$ | $OC_2H_5$ | $OC_2H_5$ | H | $NHCH_3$ |
| $CH_3$ | $CH_3$ | $CH_3$ | $CH_3$ | H | $OCH_3$ |
| $CH_3$ | $C_2H_5$ | $C_2H_5$ | $CH_3$ | H | $C_6H_5$ |
| $CH_3$ | $C(CH_3)_3$ | $C(CH_3)_3$ | $CH_3$ | H | $CH_3C_6H_4$ |
| $CH_3$ | $OCH_3$ | $OCH_3$ | $CH_3$ | H | $C_2H_5$ |
| $CH_3$ | $OC_2H_5$ | $OC_2H_5$ | $CH_3$ | H | $CH_3$ |
| $NH_2$ | Cl | $NH_2$ | Cl | H | $CH_2CH=CH_2$ |
| $NH_2$ | $NHCH_3$ | $NH_2$ | $NHCH_3$ | H | $NH_2$ |
| Br | $NHCH_3$ | Br | $NHCH_3$ | H | $NHCH_3$ |
| $NH_2$ | $CH_3$ | $NH_2$ | $CH_3$ | H | $OCH_3$ |
| Cl | $NH_2$ | Cl | $NH_2$ | H | $C_6H_5$ |
| $C_2H_5$ | $NH_2$ | $C_2H_5$ | $NH_2$ | H | $CH_3C_6H_4$ |
| $NH_2$ | $C_2H_5$ | $NH_2$ | $C_2H_5$ | H | $C_2H_5$ |
| $C_2H_5$ | $C_2H_5$ | $C_2H_5$ | $C_2H_5$ | H | $CH_3$ |
| $NH_2$ | $NH_2$ | $NH_2$ | $NH_2$ | H | $CH_2CH=CH_2$ |
| Cl | Cl | Cl | Cl | H | $NH_2$ |
| $OCH_3$ | $OCH_3$ | $OCH_3$ | $OCH_3$ | H | $NHCH_3$ |

The compound of the general formula (I) may be composed in any of a variety of manners, and may be composed, for example, by a series of reaction formula (A) or (B) set forth below:

Reaction Formula (A):

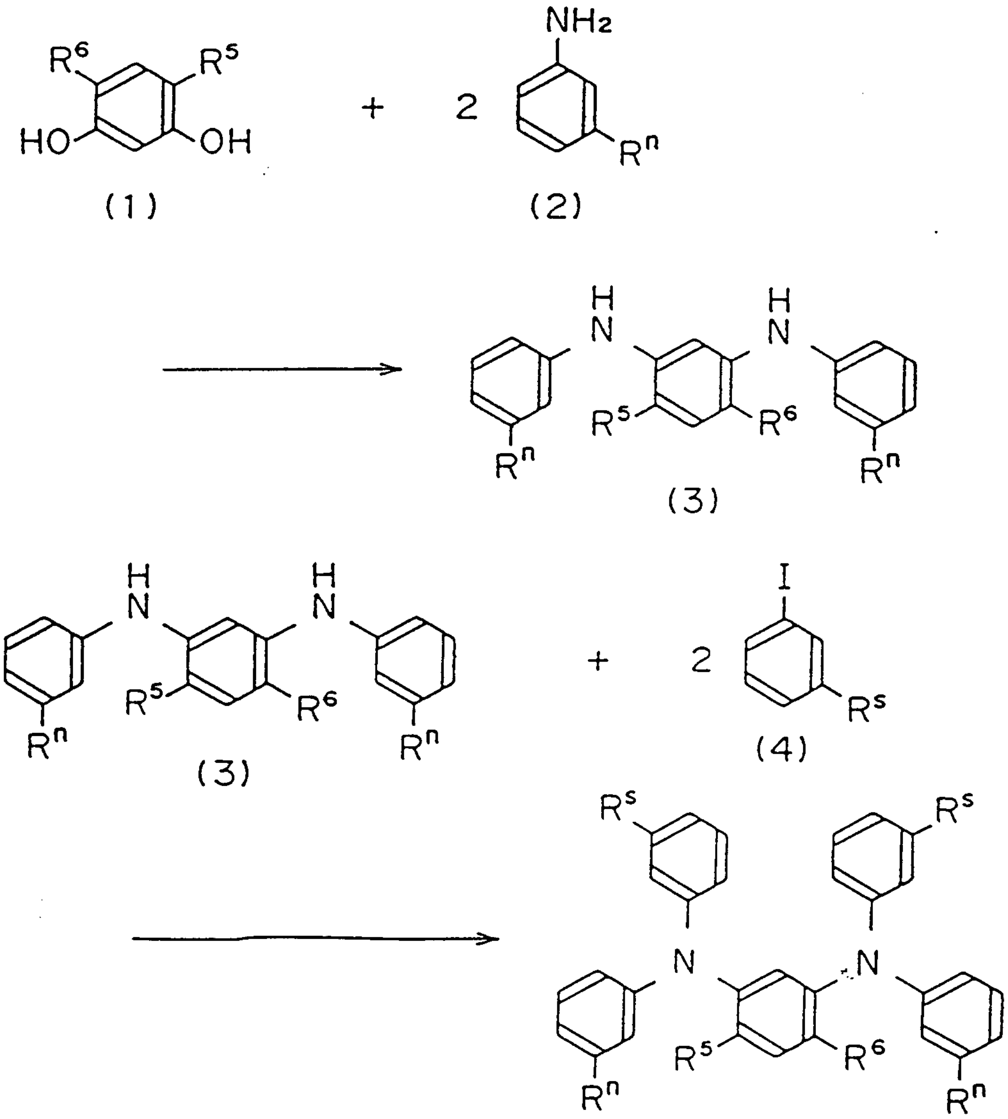

(1) (2) (3) (3) (4)

Reaction Formula (B):

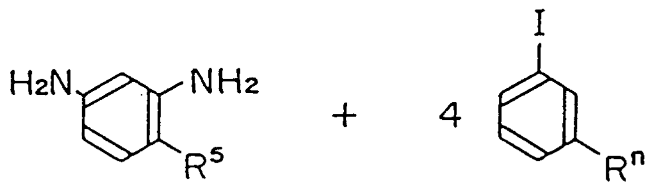

(5)

(6)

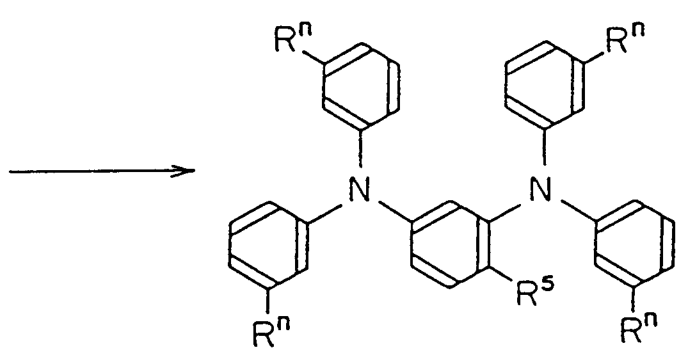

(wherein $R^5$ and $R^6$ are as defined above; $R^n$ and $R^s$ may be same as or different from each other, and each is an an alkyl group, an alkoxyl group, a halogen atom, an amino group or an N-substituting amino group).

In the reaction formula (A), a resorcinol derivative represented by the formula (1) and an aniline derivative represented by the formula (2) are reacted at reflux in a stream of nitrogen to give a phenylenediamine compound represented by the formula (3). Then, the compound of the formula (3) and an iodobenzene derivative represented by the formula (4) are reacted in a solvent (for example, nitrobenzene) in the presence of potassium carbonate and copper powder to give the m-phenylenediamine compound [I] of the present invention in which $R^2$ and $R^4$ are same as each other and $R^1$ and $R^3$ are same as each other in the general formula (I).

As above-mentioned, the substituting groups $R^5$ and $R^6$ such as the alkyl group or the like may be previously introduced in resorcinol, for example. $R^n$ (which is $R^2$ or $R^4$ in the general formula (I)) may be previously introduced in aniline, while the substituting group $R^s$ (which is $R^1$ or $R^3$ in the general formula (I)) may be previously introduced in iodobenzene.

On the other hand, as shown in the reaction formula (B), a phenylenediamine derivative of the formula (5) and an iodobenzene derivative of the formula (6) are reacted at reflux in a solvent (for example, nitrobenzene) in the presence of potassium carbonate and copper powder, thereby to compose the m-phenylenediamine compound (I) in which $R^1$ to $R^4$ are the same substituting group in the general formula (I).

To compose other compound of the present invention than the compound obtainable by the reaction formula (A) or (B), e.g., a compound in which all $R^1$ to $R^4$ are respectively different substituting groups, there is proposed a method by which the molecular ratio of the starting materials in the reaction formula (A) or (B) is suitably adjusted and phenyl groups having substituting groups $R^1$ to $R^4$ are gradually introduced. Alternately, after a phenyl group having no substituting group has been introduced, the substituting groups $R^1$ to $R^4$, $R^5$ and $R^6$ may be successively introduced.

As specific examples of the m-phenylenediamine compound represented by the general formula (II), the following compounds may be mentioned.

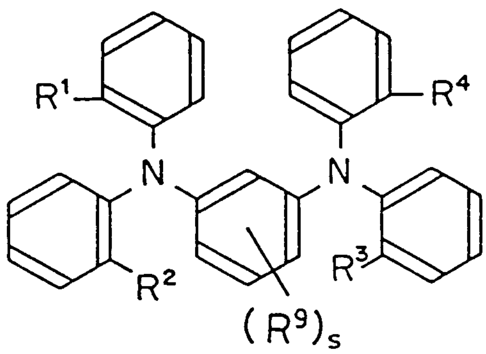

(II)

| $R^1$ | $R^2$ | $R^3$ | $R^4$ | $R^9$ |
|---|---|---|---|---|
| $OC_2H_5$ | $CH_3$ | $CH_3$ | $OC_2H_5$ | $CH_3$ |
| $C_2H_5$ | $C_2H_5$ | $C_2H_5$ | $C_2H_5$ | $CH_2CH=CH_2$ |
| $C_3H_7$ | $C_3H_7$ | $C_3H_7$ | $C_3H_7$ | $NH_2$ |
| $C(CH_3)_3$ | $C(CH_3)_3$ | $C(CH_3)_3$ | $C(CH_3)_3$ | $NHCH_3$ |
| $OCH_3$ | $OCH_3$ | $OCH_3$ | $OCH_3$ | $OCH_3$ |
| $OC_2H_5$ | $OC_2H_5$ | $OC_2H_5$ | $OC_2H_5$ | $CH_3$ |
| $C_2H_5$ | $CH_3$ | $CH_3$ | $C_2H_5$ | $CH_2CH=CH_2$ |
| $C(CH_3)_3$ | $CH_3$ | $CH_3$ | $C(CH_3)_3$ | $NHCH_3$ |
| $OCH_3$ | $CH_3$ | $CH_3$ | $OCH_3$ | $OCH_3$ |

| $R^1$ | $R^2$ | $R^3$ | $R^4$ | $R^9$ |
|---|---|---|---|---|
| $CH_3$ | $CH_3$ | $CH_3$ | $CH_3$ | $C_6H_5$ |
| $CH_3$ | $CH_3$ | $CH_3$ | $CH_3$ | $NH_2$ |
| $CH_3$ | $CH_3$ | $CH_3$ | $CH_3$ | $NH_2$ |
| $CH_3$ | $CH_3$ | $CH_3$ | $CH_3$ | $NHCH_3$ |
| $CH_3$ | $CH_3$ | $CH_3$ | $CH_3$ | $OCH_3$ |
| $CH_3$ | $CH_3$ | $CH_3$ | $CH_3$ | $CH_3$ |
| $CH_3$ | $C_2H_5$ | $C_2H_5$ | $CH_3$ | $C_6H_5$ |
| $CH_3$ | $C_3H_7$ | $C_3H_7$ | $CH_3$ | $C_6H_5$ |
| $CH_3$ | $C(CH_3)_3$ | $C(CH_3)_3$ | $CH_3$ | $C_6H_5$ |
| $CH_3$ | $OCH_3$ | $OCH_3$ | $CH_3$ | $C_6H_5$ |
| $CH_3$ | $OC_2H_5$ | $OC_2H_5$ | $CH_3$ | $C_6H_5$ |
| $C_2H_5$ | $CH_3$ | $CH_3$ | $C_2H_5$ | $C_6H_5$ |
| $C_3H_7$ | $CH_3$ | $CH_3$ | $C_3H_7$ | $CH_3C_6H_4$ |
| $C(CH_3)_3$ | $CH_3$ | $CH_3$ | $C(CH_3)_3$ | $CH_3C_6H_4$ |
| $OCH_3$ | $CH_3$ | $CH_3$ | $OCH_3$ | $CH_3C_6H_4$ |

| $R^1$ | $R^2$ | $R^3$ | $R^4$ | $R^9$ |
|---|---|---|---|---|
| $OC_2H_5$ | $CH_3$ | $CH_3$ | $OC_2H_5$ | $C_6H_5$ |
| $CH_3$ | $C_2H_5$ | $C_2H_5$ | $CH_3$ | $CH_2CH=CH_2$ |
| $CH_3$ | $C_3H_7$ | $C_3H_7$ | $CH_3$ | $CH_2CH=CH_2$ |
| $CH_3$ | $C(CH_3)_3$ | $C(CH_3)_3$ | $CH_3$ | $CH_2CH=CH_2$ |
| $CH_3$ | $OCH_3$ | $OCH_3$ | $CH_3$ | $CH_2CH=CH_2$ |
| $CH_3$ | $OC_2H_5$ | $OC_2H_5$ | $CH_3$ | $CH_2CH=CH_2$ |
| $C_2H_5$ | $CH_3$ | $CH_3$ | $C_2H_5$ | $CH_2CH=CH_2$ |
| $C_3H_7$ | $CH_3$ | $CH_3$ | $C_3H_7$ | $CH_2CH=CH_2$ |
| $C(CH_3)_3$ | $CH_3$ | $CH_3$ | $C(CH_3)_3$ | $CH_2CH=CH_2$ |
| $OCH_3$ | $CH_3$ | $CH_3$ | $OCH_3$ | $CH_2CH=CH_2$ |
| $OC_2H_5$ | $CH_3$ | $CH_3$ | $OC_2H_5$ | $CH_2CH=CH_2$ |
| $CH_3$ | $C_2H_5$ | $C_2H_5$ | $CH_3$ | $NH_2$ |
| $CH_3$ | $C_3H_7$ | $C_3H_7$ | $CH_3$ | $NH_2$ |
| $CH_3$ | $C(CH_3)_3$ | $C(CH_3)_3$ | $CH_3$ | $NH_2$ |
| $CH_3$ | $OCH_3$ | $OCH_3$ | $CH_3$ | $NH_2$ |

| $R^1$ | $R^2$ | $R^3$ | $R^4$ | $R^9$ |
|---|---|---|---|---|
| $CH_3$ | $OC_2H_5$ | $OC_2H_5$ | $CH_3$ | $NH_2$ |
| $C_2H_5$ | $CH_3$ | $CH_3$ | $C_2H_5$ | $NH_2$ |
| $C_3H_7$ | $CH_3$ | $CH_3$ | $C_3H_7$ | $NH_2$ |
| $C(CH_3)_3$ | $CH_3$ | $CH_3$ | $C(CH_3)_3$ | $NH_2$ |
| $OCH_3$ | $CH_3$ | $CH_3$ | $OCH_3$ | $NH_2$ |
| $OC_2H_5$ | $CH_3$ | $CH_3$ | $OC_2H_5$ | $NH_2$ |
| $CH_3$ | $C_2H_5$ | $C_2H_5$ | $CH_3$ | $NHCH_3$ |
| $CH_3$ | $C(CH_3)_3$ | $C(CH_3)_3$ | $CH_3$ | $NHCH_3$ |
| $CH_3$ | $OCH_3$ | $OCH_3$ | $CH_3$ | $NHCH_3$ |
| $CH_3$ | $OC_2H_5$ | $OC_2H_5$ | $CH_3$ | $NHCH_3$ |
| $C_2H_5$ | $CH_3$ | $CH_3$ | $C_2H_5$ | $NHCH_3$ |
| $C(CH_3)_3$ | $CH_3$ | $CH_3$ | $C(CH_3)_3$ | $NHCH_3$ |
| $OCH_3$ | $CH_3$ | $CH_3$ | $OCH_3$ | $NHCH_3$ |
| $OC_2H_5$ | $CH_3$ | $CH_3$ | $OC_2H_5$ | $NHCH_3$ |

| $R^1$ | $R^2$ | $R^3$ | $R^4$ | $R^9$ |
|---|---|---|---|---|
| $CH_3$ | $C_2H_5$ | $C_2H_5$ | $CH_3$ | $OCH_3$ |
| $CH_3$ | $C(CH_3)_3$ | $C(CH_3)_3$ | $CH_3$ | $OCH_3$ |
| $CH_3$ | $OCH_3$ | $OCH_3$ | $CH_3$ | $OCH_3$ |
| $CH_3$ | $OC_2H_5$ | $OC_2H_5$ | $CH_3$ | $OCH_3$ |
| $C_2H_5$ | $CH_3$ | $CH_3$ | $C_2H_5$ | $OCH_3$ |
| $C(CH_3)_3$ | $CH_3$ | $CH_3$ | $C(CH_3)_3$ | $OCH_3$ |
| $OCH_3$ | $CH_3$ | $CH_3$ | $OCH_3$ | $OCH_3$ |
| $OC_2H_5$ | $CH_3$ | $CH_3$ | $OC_2H_5$ | $OCH_3$ |
| $CH_3$ | $C_2H_5$ | $C_2H_5$ | $CH_3$ | $CH_3$ |
| $CH_3$ | $C(CH_3)_3$ | $C(CH_3)_3$ | $CH_3$ | $CH_3$ |
| $CH_3$ | $OCH_3$ | $OCH_3$ | $CH_3$ | $CH_3$ |
| $CH_3$ | $OC_2H_5$ | $OC_2H_5$ | $CH_3$ | $CH_3$ |

| $R^1$ | $R^2$ | $R^3$ | $R^4$ | $R^9$ |
|---|---|---|---|---|
| Cl | Cl | Cl | Cl | Cl |
| $NH_2$ | $NH_2$ | $NH_2$ | $NH_2$ | $NH_2$ |
| $NHCH_3$ | $NHCH_3$ | $NHCH_3$ | $NHCH_3$ | $NHCH_3$ |
| $NH_2$ | Cl | $NH_2$ | Cl | $CH_3$ |
| $NH_2$ | Cl | $NH_2$ | Cl | $CH_2CH=CH_2$ |
| $NH_2$ | Cl | $NH_2$ | Cl | $NH_2$ |
| $NH_2$ | Cl | $NH_2$ | Cl | $NHCH_3$ |
| $NH_2$ | Cl | $NH_2$ | Cl | $OCH_3$ |
| $NH_2$ | Cl | $NH_2$ | Cl | $C_6H_5$ |
| $NH_2$ | Cl | $NH_2$ | Cl | $CH_3C_6H_4$ |
| $NH_2$ | $NHCH_3$ | $NH_2$ | $NHCH_3$ | $CH_3$ |
| $NH_2$ | $NHCH_3$ | $NH_2$ | $NHCH_3$ | $CH_2CH=CH_2$ |
| $NH_2$ | $NHCH_3$ | $NH_2$ | $NHCH_3$ | $NH_2$ |
| $NH_2$ | $NHCH_3$ | $NH_2$ | $NHCH_3$ | $NHCH_3$ |
| $NH_2$ | $NHCH_3$ | $NH_2$ | $NHCH_3$ | $OCH_3$ |
| $NH_2$ | $NHCH_3$ | $NH_2$ | $NHCH_3$ | $C_6H_5$ |
| Br | $NHCH_3$ | Br | $NHCH_3$ | $CH_3$ |
| Br | $NHCH_3$ | Br | $NHCH_3$ | $CH_2CH=CH_2$ |

| $R^1$ | $R^2$ | $R^3$ | $R^4$ | $R^9$ |
|---|---|---|---|---|
| Br | $NHCH_3$ | Br | $NHCH_3$ | $NH_2$ |
| Br | $NHCH_3$ | Br | $NHCH_3$ | $NHCH_3$ |
| Br | $NHCH_3$ | Br | $NHCH_3$ | $OCH_3$ |
| Br | $NHCH_3$ | Br | $NHCH_3$ | $C_6H_5$ |
| Br | $NHCH_3$ | Br | $NHCH_3$ | $CH_3C_6H_4$ |
| Br | $NHCH_3$ | Br | $NHCH_3$ | $C_2H_5$ |
| $NH_2$ | $CH_3$ | $NH_2$ | $CH_3$ | $CH_3$ |
| $NH_2$ | $CH_3$ | $NH_2$ | $CH_3$ | $CH_2CH=CH_2$ |
| $NH_2$ | $CH_3$ | $NH_2$ | $CH_3$ | $NH_2$ |
| $NH_2$ | $CH_3$ | $NH_2$ | $CH_3$ | $NHCH_3$ |
| $NH_2$ | $CH_3$ | $NH_2$ | $CH_3$ | $OCH_3$ |
| $NH_2$ | $CH_3$ | $NH_2$ | $CH_3$ | $C_6H_5$ |
| $NH_2$ | $CH_3$ | $NH_2$ | $CH_3$ | $CH_3C_6H_4$ |
| $NH_2$ | $CH_3$ | $NH_2$ | $CH_3$ | $C_2H_5$ |
| Cl | $NH_2$ | Cl | $NH_2$ | $CH_3$ |
| Cl | $NH_2$ | Cl | $NH_2$ | $CH_2CH=CH_2$ |
| Cl | $NH_2$ | Cl | $NH_2$ | $NH_2$ |
| Cl | $NH_2$ | Cl | $NH_2$ | $NHCH_3$ |

| $R^1$ | $R^2$ | $R^3$ | $R^4$ | $R^9$ |
|---|---|---|---|---|
| $OC_2H_5$ | $CH_3$ | $CH_3$ | $OC_2H_5$ | H |
| $OC_2H_5$ | $OC_2H_5$ | $OC_2H_5$ | $OC_2H_5$ | H |
| $CH_3$ | $CH_3$ | $CH_3$ | $CH_3$ | H |
| $CH_3$ | $C_2H_5$ | $C_2H_5$ | $CH_3$ | H |
| $CH_3$ | $C(CH_3)_3$ | $C(CH_3)_3$ | $CH_3$ | H |
| $CH_3$ | $OCH_3$ | $OCH_3$ | $CH_3$ | H |
| $CH_3$ | $OC_2H_5$ | $OC_2H_5$ | $CH_3$ | H |
| $NH_2$ | Cl | $NH_2$ | Cl | H |
| $NH_2$ | $NHCH_3$ | $NH_2$ | $NHCH_3$ | H |
| Br | $NHCH_3$ | Br | $NHCH_3$ | H |
| $NH_2$ | $CH_3$ | $NH_2$ | $CH_3$ | H |
| Cl | $NH_2$ | Cl | $NH_2$ | H |
| $C_2H_5$ | $NH_2$ | $C_2H_5$ | $NH_2$ | H |
| $NH_2$ | $C_2H_5$ | $NH_2$ | $C_2H_5$ | H |
| $C_2H_5$ | $C_2H_5$ | $C_2H_5$ | $C_2H_5$ | H |
| $NH_2$ | $NH_2$ | $NH_2$ | $NH_2$ | H |
| Cl | Cl | Cl | Cl | H |
| $OCH_3$ | $OCH_3$ | $OCH_3$ | $OCH_3$ | H |

The compound of the general formula (II) may be composed in any of a variety of manners, and may be composed, for example, by a series of reaction formula (C) or (D) set forth below:

Reaction Formula (C):

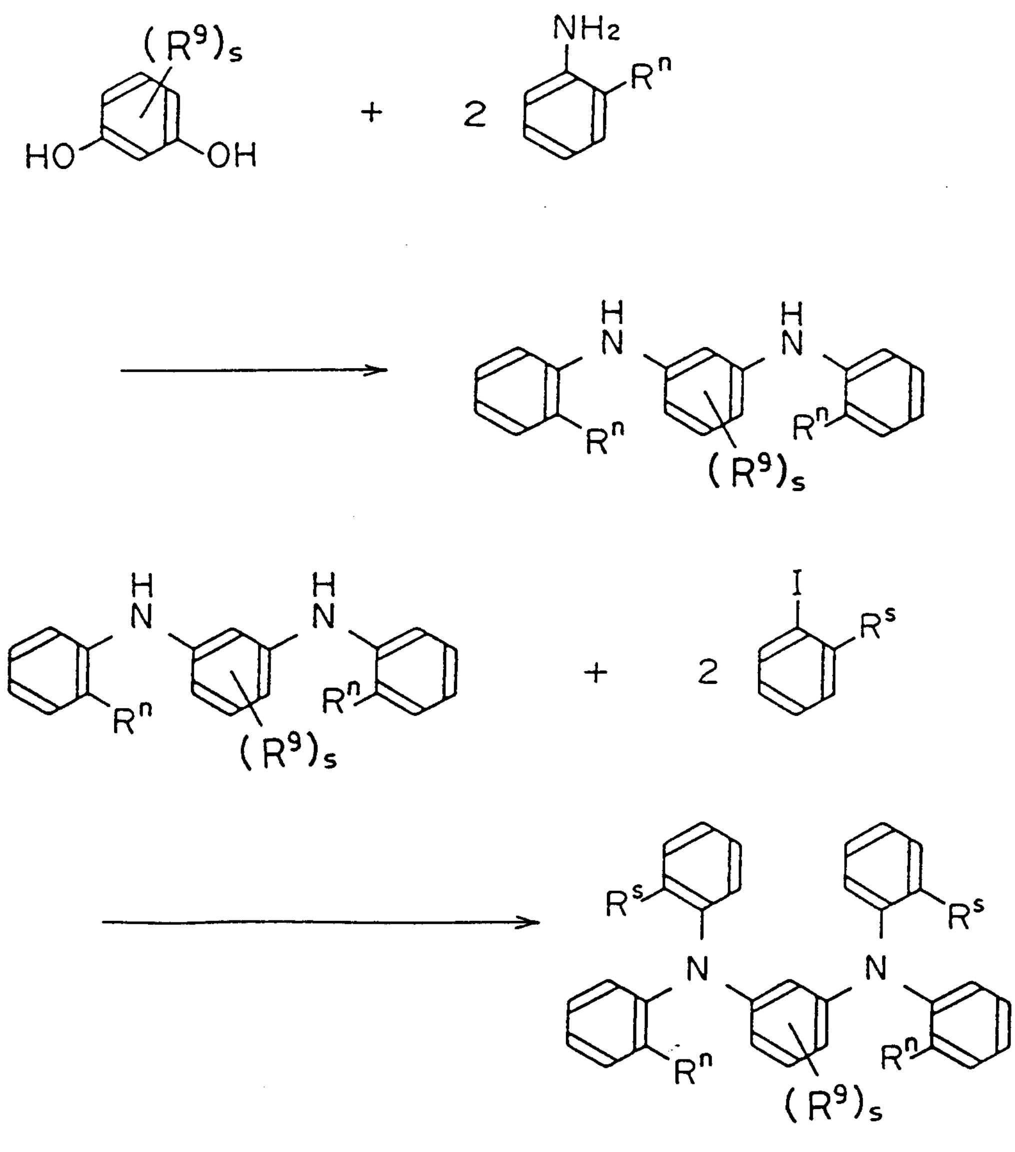

Reaction Formula (D):

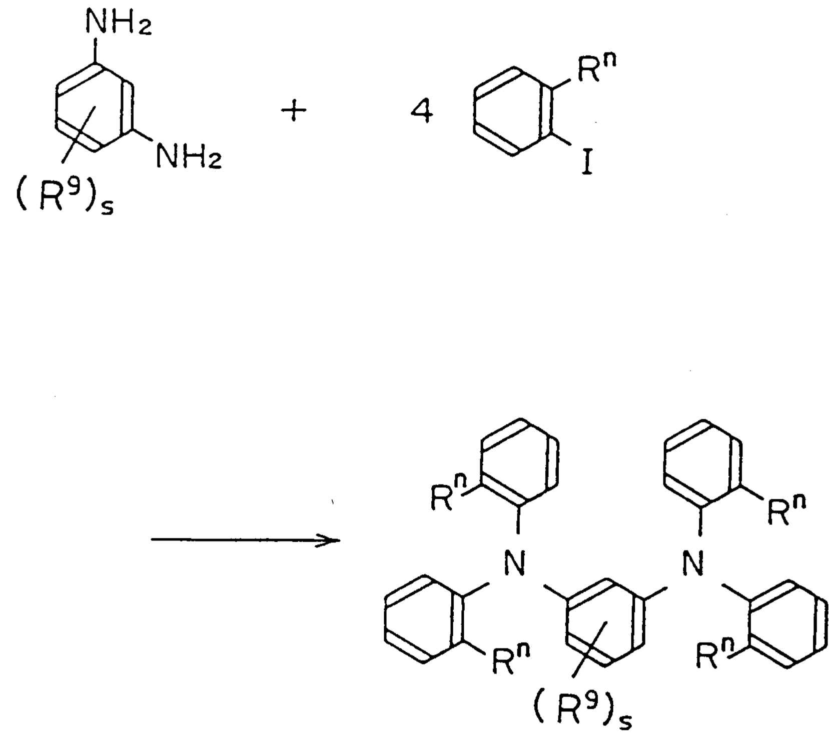

(wherein $R^9$ and s are as defined above; $R^n$ and $R^s$ may be same as or different from each other, and each is an an alkyl group, an alkoxyl group, a halogen atom, an amino group or an N-substituting amino group).

The reactions of the formulas (C) and (D) may be conducted under the same conditions as those for the reactions of the formulas (A) and (B). Accordingly, the detailed description of the reactions of the formulas (C) and (D) will be here omitted.

As specific examples of the m-phenylenediamine compound represented by the general formula (III), the following compounds may be mentioned.

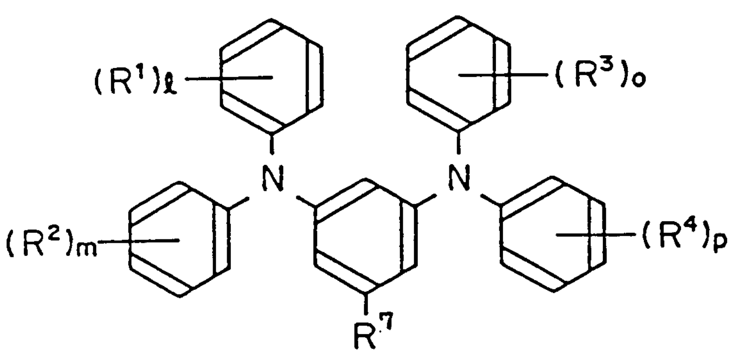

(III)

| $R^1$ | $R^2$ | $R^3$ | $R^4$ | $R^7$ |
|---|---|---|---|---|
| $OC_2H_5$ | $CH_3$ | $CH_3$ | $OC_2H_5$ | $CH_3$ |
| $C_2H_5$ | $C_2H_5$ | $C_2H_5$ | $C_2H_5$ | $CH_2CH=CH_2$ |
| $C_3H_7$ | $C_3H_7$ | $C_3H_7$ | $C_3H_7$ | $NH_2$ |
| $C(CH_3)_3$ | $C(CH_3)_3$ | $C(CH_3)_3$ | $C(CH_3)_3$ | $NHCH_3$ |
| $OCH_3$ | $OCH_3$ | $OCH_3$ | $OCH_3$ | $OCH_3$ |
| $OC_2H_5$ | $OC_2H_5$ | $OC_2H_5$ | $OC_2H_5$ | $CH_3$ |
| H | $CH_3$ | $CH_3$ | H | $NH_2$ |
| $C_2H_5$ | $CH_3$ | $CH_3$ | $C_2H_5$ | $CH_2CH=CH_2$ |
| $C(CH_3)_3$ | $CH_3$ | $CH_3$ | $C(CH_3)_3$ | $NHCH_3$ |
| $OCH_3$ | $CH_3$ | $CH_3$ | $OCH_3$ | $OCH_3$ |

| $R^1$ | $R^2$ | $R^3$ | $R^4$ | $R^7$ |
|---|---|---|---|---|
| H | H | H | H | $NH_2$ |
| $CH_3$ | $CH_3$ | $CH_3$ | $CH_3$ | $C_6H_5$ |
| $CH_3$ | $CH_3$ | $CH_3$ | $CH_3$ | $NH_2$ |
| $CH_3$ | $CH_3$ | $CH_3$ | $CH_3$ | $NH_2$ |
| $CH_3$ | $CH_3$ | $CH_3$ | $CH_3$ | $NHCH_3$ |
| $CH_3$ | $CH_3$ | $CH_3$ | $CH_3$ | $OCH_3$ |
| $CH_3$ | $CH_3$ | $CH_3$ | $CH_3$ | $CH_3$ |
| $CH_3$ | H | H | $CH_3$ | $C_6H_5$ |
| $CH_3$ | $C_2H_5$ | $C_2H_5$ | $CH_3$ | $C_6H_5$ |
| $CH_3$ | $C_3H_7$ | $C_3H_7$ | $CH_3$ | $C_6H_5$ |
| $CH_3$ | $C(CH_3)_3$ | $C(CH_3)_3$ | $CH_3$ | $C_6H_5$ |
| $CH_3$ | $OCH_3$ | $OCH_3$ | $CH_3$ | $C_6H_5$ |
| $CH_3$ | $OC_2H_5$ | $OC_2H_5$ | $CH_3$ | $C_6H_5$ |
| H | $CH_3$ | $CH_3$ | H | $C_6H_5$ |
| $C_2H_5$ | $CH_3$ | $CH_3$ | $C_2H_5$ | $C_6H_5$ |
| $C_3H_7$ | $CH_3$ | $CH_3$ | $C_3H_7$ | $CH_3C_6H_4$ |
| $C(CH_3)_3$ | $CH_3$ | $CH_3$ | $C(CH_3)_3$ | $CH_3C_6H_4$ |
| $OCH_3$ | $CH_3$ | $CH_3$ | $OCH_3$ | $CH_3C_6H_4$ |

| $R^1$ | $R^2$ | $R^3$ | $R^4$ | $R^7$ |
|---|---|---|---|---|
| $OC_2H_5$ | $CH_3$ | $CH_3$ | $OC_2H_5$ | $C_6H_5$ |
| $CH_3$ | H | H | $CH_3$ | $CH_2CH=CH_2$ |
| $CH_3$ | $C_2H_5$ | $C_2H_5$ | $CH_3$ | $CH_2CH=CH_2$ |
| $CH_3$ | $C_3H_7$ | $C_3H_7$ | $CH_3$ | $CH_2CH=CH_2$ |
| $CH_3$ | $C(CH_3)_3$ | $C(CH_3)_3$ | $CH_3$ | $CH_2CH=CH_2$ |
| $CH_3$ | $OCH_3$ | $OCH_3$ | $CH_3$ | $CH_2CH=CH_2$ |
| $CH_3$ | $OC_2H_5$ | $OC_2H_5$ | $CH_3$ | $CH_2CH=CH_2$ |
| H | $CH_3$ | $CH_3$ | H | $CH_2CH=CH_2$ |
| $C_2H_5$ | $CH_3$ | $CH_3$ | $C_2H_5$ | $CH_2CH=CH_2$ |
| $C_3H_7$ | $CH_3$ | $CH_3$ | $C_3H_7$ | $CH_2CH=CH_2$ |
| $C(CH_3)_3$ | $CH_3$ | $CH_3$ | $C(CH_3)_3$ | $CH_2CH=CH_2$ |
| $OCH_3$ | $CH_3$ | $CH_3$ | $OCH_3$ | $CH_2CH=CH_2$ |
| $OC_2H_5$ | $CH_3$ | $CH_3$ | $OC_2H_5$ | $CH_2CH=CH_2$ |
| $CH_3$ | H | H | $CH_3$ | $NH_2$ |
| $CH_3$ | $C_2H_5$ | $C_2H_5$ | $CH_3$ | $NH_2$ |
| $CH_3$ | $C_3H_7$ | $C_3H_7$ | $CH_3$ | $NH_2$ |
| $CH_3$ | $C(CH_3)_3$ | $C(CH_3)_3$ | $CH_3$ | $NH_2$ |
| $CH_3$ | $OCH_3$ | $OCH_3$ | $CH_3$ | $NH_2$ |

| $R^1$ | $R^2$ | $R^3$ | $R^4$ | $R^7$ |
|---|---|---|---|---|
| $CH_3$ | $OC_2H_5$ | $OC_2H_5$ | $CH_3$ | $NH_2$ |
| H | $CH_3$ | $CH_3$ | H | $NH_2$ |
| $C_2H_5$ | $CH_3$ | $CH_3$ | $C_2H_5$ | $NH_2$ |
| $C_3H_7$ | $CH_3$ | $CH_3$ | $C_3H_7$ | $NH_2$ |
| $C(CH_3)_3$ | $CH_3$ | $CH_3$ | $C(CH_3)_3$ | $NH_2$ |
| $OCH_3$ | $CH_3$ | $CH_3$ | $OCH_3$ | $NH_2$ |
| $OC_2H_5$ | $CH_3$ | $CH_3$ | $OC_2H_5$ | $NH_2$ |
| $CH_3$ | H | H | $CH_3$ | $NHCH_3$ |
| $CH_3$ | $C_2H_5$ | $C_2H_5$ | $CH_3$ | $NHCH_3$ |
| $CH_3$ | $C(CH_3)_3$ | $C(CH_3)_3$ | $CH_3$ | $NHCH_3$ |
| $CH_3$ | $OCH_3$ | $OCH_3$ | $CH_3$ | $NHCH_3$ |
| $CH_3$ | $OC_2H_5$ | $OC_2H_5$ | $CH_3$ | $NHCH_3$ |
| H | $CH_3$ | $CH_3$ | H | $NHCH_3$ |
| $C_2H_5$ | $CH_3$ | $CH_3$ | $C_2H_5$ | $NHCH_3$ |
| $C(CH_3)_3$ | $CH_3$ | $CH_3$ | $C(CH_3)_3$ | $NHCH_3$ |
| $OCH_3$ | $CH_3$ | $CH_3$ | $OCH_3$ | $NHCH_3$ |
| $OC_2H_5$ | $CH_3$ | $CH_3$ | $OC_2H_5$ | $NHCH_3$ |
| $CH_3$ | H | H | $CH_3$ | $OCH_3$ |

| $R^1$ | $R^2$ | $R^3$ | $R^4$ | $R^7$ |
|---|---|---|---|---|
| $CH_3$ | $C_2H_5$ | $C_2H_5$ | $CH_3$ | $OCH_3$ |
| $CH_3$ | $C(CH_3)_3$ | $C(CH_3)_3$ | $CH_3$ | $OCH_3$ |
| $CH_3$ | $OCH_3$ | $OCH_3$ | $CH_3$ | $OCH_3$ |
| $CH_3$ | $OC_2H_5$ | $OC_2H_5$ | $CH_3$ | $OCH_3$ |
| H | $CH_3$ | $CH_3$ | H | $OCH_3$ |
| $C_2H_5$ | $CH_3$ | $CH_3$ | $C_2H_5$ | $OCH_3$ |
| $C(CH_3)_3$ | $CH_3$ | $CH_3$ | $C(CH_3)_3$ | $OCH_3$ |
| $OCH_3$ | $CH_3$ | $CH_3$ | $OCH_3$ | $OCH_3$ |
| $OC_2H_5$ | $CH_3$ | $CH_3$ | $OC_2H_5$ | $OCH_3$ |
| $CH_3$ | H | H | $CH_3$ | $CH_3$ |
| $CH_3$ | $C_2H_5$ | $C_2H_5$ | $CH_3$ | $CH_3$ |
| $CH_3$ | $C(CH_3)_3$ | $C(CH_3)_3$ | $CH_3$ | $CH_3$ |
| $CH_3$ | $OCH_3$ | $OCH_3$ | $CH_3$ | $CH_3$ |
| $CH_3$ | $OC_2H_5$ | $OC_2H_5$ | $CH_3$ | $CH_3$ |

| $R^1$ | $R^2$ | $R^3$ | $R^4$ | $R^7$ |
|---|---|---|---|---|
| Cl | Cl | Cl | Cl | Cl |
| $NH_2$ | $NH_2$ | $NH_2$ | $NH_2$ | $NH_2$ |
| $NHCH_3$ | $NHCH_3$ | $NHCH_3$ | $NHCH_3$ | $NHCH_3$ |
| $NH_2$ | Cl | $NH_2$ | Cl | $CH_3$ |
| $NH_2$ | Cl | $NH_2$ | Cl | $CH_2CH=CH_2$ |
| $NH_2$ | Cl | $NH_2$ | Cl | $NH_2$ |
| $NH_2$ | Cl | $NH_2$ | Cl | $NHCH_3$ |
| $NH_2$ | Cl | $NH_2$ | Cl | $OCH_3$ |
| $NH_2$ | Cl | $NH_2$ | Cl | $C_6H_5$ |
| $NH_2$ | Cl | $NH_2$ | Cl | $CH_3C_6H_4$ |
| $NH_2$ | $NHCH_3$ | $NH_2$ | $NHCH_3$ | $CH_3$ |
| $NH_2$ | $NHCH_3$ | $NH_2$ | $NHCH_3$ | $CH_2CH=CH_2$ |
| $NH_2$ | $NHCH_3$ | $NH_2$ | $NHCH_3$ | $NH_2$ |
| $NH_2$ | $NHCH_3$ | $NH_2$ | $NHCH_3$ | $NHCH_3$ |
| $NH_2$ | $NHCH_3$ | $NH_2$ | $NHCH_3$ | $OCH_3$ |
| $NH_2$ | $NHCH_3$ | $NH_2$ | $NHCH_3$ | $C_6H_5$ |
| Br | $NHCH_3$ | Br | $NHCH_3$ | $CH_3$ |
| Br | $NHCH_3$ | Br | $NHCH_3$ | $CH_2CH=CH_2$ |

| $R^1$ | $R^2$ | $R^3$ | $R^4$ | $R^7$ |
|---|---|---|---|---|
| Br | $NHCH_3$ | Br | $NHCH_3$ | $NH_2$ |
| Br | $NHCH_3$ | Br | $NHCH_3$ | $NHCH_3$ |
| Br | $NHCH_3$ | Br | $NHCH_3$ | $OCH_3$ |
| Br | $NHCH_3$ | Br | $NHCH_3$ | $C_6H_5$ |
| Br | $NHCH_3$ | Br | $NHCH_3$ | $CH_3C_6H_4$ |
| Br | $NHCH_3$ | Br | $NHCH_3$ | $C_2H_5$ |
| $NH_2$ | $CH_3$ | $NH_2$ | $CH_3$ | $CH_3$ |
| $NH_2$ | $CH_3$ | $NH_2$ | $CH_3$ | $CH_2CH=CH_2$ |
| $NH_2$ | $CH_3$ | $NH_2$ | $CH_3$ | $NH_2$ |
| $NH_2$ | $CH_3$ | $NH_2$ | $CH_3$ | $NHCH_3$ |
| $NH_2$ | $CH_3$ | $NH_2$ | $CH_3$ | $OCH_3$ |
| $NH_2$ | $CH_3$ | $NH_2$ | $CH_3$ | $C_6H_5$ |
| $NH_2$ | $CH_3$ | $NH_2$ | $CH_3$ | $CH_3C_6H_4$ |
| $NH_2$ | $CH_3$ | $NH_2$ | $CH_3$ | $C_2H_5$ |
| Cl | $NH_2$ | Cl | $NH_2$ | $CH_3$ |
| Cl | $NH_2$ | Cl | $NH_2$ | $CH_2CH=CH_2$ |
| Cl | $NH_2$ | Cl | $NH_2$ | $NH_2$ |
| Cl | $NH_2$ | Cl | $NH_2$ | $NHCH_3$ |

| $R^1$ | $R^2$ | $R^3$ | $R^4$ | $R^7$ |
|---|---|---|---|---|
| $OC_2H_5$ | $CH_3$ | $CH_3$ | $OC_2H_5$ | $NO_2$ |
| $C_2H_5$ | $C_2H_5$ | $C_2H_5$ | $C_2H_5$ | $SO_3H$ |
| $C_3H_7$ | $C_3H_7$ | $C_3H_7$ | $C_3H_7$ | CN |
| $C(CH_3)_3$ | $C(CH_3)_3$ | $C(CH_3)_3$ | $C(CH_3)_3$ | CHO |
| $OCH_3$ | $OCH_3$ | $OCH_3$ | $OCH_3$ | Cl |
| $OC_2H_5$ | $OC_2H_5$ | $OC_2H_5$ | $OC_2H_5$ | COOH |
| H | $CH_3$ | $CH_3$ | H | $NO_2$ |
| $C_2H_5$ | $CH_3$ | $CH_3$ | $C_2H_5$ | $NO_2$ |
| $C(CH_3)_3$ | $CH_3$ | $CH_3$ | $C(CH_3)_3$ | COOH |
| $OCH_3$ | $CH_3$ | $CH_3$ | $OCH_3$ | COOH |

| $R^1$ | $R^2$ | $R^3$ | $R^4$ | $R^7$ |
|---|---|---|---|---|
| H | H | H | H | $NO_2$ |
| $CH_3$ | $CH_3$ | $CH_3$ | $CH_3$ | $COCH_3$ |
| $CH_3$ | $CH_3$ | $CH_3$ | $CH_3$ | $SO_3H$ |
| $CH_3$ | $CH_3$ | $CH_3$ | $CH_3$ | CN |
| $CH_3$ | $CH_3$ | $CH_3$ | $CH_3$ | CHO |
| $CH_3$ | $CH_3$ | $CH_3$ | $CH_3$ | Cl |
| $CH_3$ | $CH_3$ | $CH_3$ | $CH_3$ | COOH |
| $CH_3$ | H | H | $CH_3$ | $COCH_3$ |
| $CH_3$ | $C_2H_5$ | $C_2H_5$ | $CH_3$ | $COCH_3$ |
| $CH_3$ | $C_3H_7$ | $C_3H_7$ | $CH_3$ | $COC_2H_5$ |
| $CH_3$ | $C(CH_3)_3$ | $C(CH_3)_3$ | $CH_3$ | $COC_2H_5$ |
| $CH_3$ | $OCH_3$ | $OCH_3$ | $CH_3$ | $COC_2H_5$ |
| $CH_3$ | $OC_2H_5$ | $OC_2H_5$ | $CH_3$ | $COC_2H_5$ |
| H | $CH_3$ | $CH_3$ | H | $COCH_3$ |
| $C_2H_5$ | $CH_3$ | $CH_3$ | $C_2H_5$ | $COCH_3$ |
| $C_3H_7$ | $CH_3$ | $CH_3$ | $C_3H_7$ | $COCH_3$ |
| $C(CH_3)_3$ | $CH_3$ | $CH_3$ | $C(CH_3)_3$ | $COC_2H_5$ |
| $OCH_3$ | $CH_3$ | $CH_3$ | $OCH_3$ | $COC_2H_5$ |

| $R^1$ | $R^2$ | $R^3$ | $R^4$ | $R^7$ |
|---|---|---|---|---|
| $OC_2H_5$ | $CH_3$ | $CH_3$ | $OC_2H_5$ | $COCH_3$ |
| $CH_3$ | H | H | $CH_3$ | $SO_3H$ |
| $CH_3$ | $C_2H_5$ | $C_2H_5$ | $CH_3$ | $SO_3H$ |
| $CH_3$ | $C_3H_7$ | $C_3H_7$ | $CH_3$ | $SO_3H$ |
| $CH_3$ | $C(CH_3)_3$ | $C(CH_3)_3$ | $CH_3$ | $SO_3H$ |
| $CH_3$ | $OCH_3$ | $OCH_3$ | $CH_3$ | $COOCH_3$ |
| $CH_3$ | $OC_2H_5$ | $OC_2H_5$ | $CH_3$ | $COOCH_3$ |
| H | $CH_3$ | $CH_3$ | H | $COOCH_3$ |
| $C_2H_5$ | $CH_3$ | $CH_3$ | $C_2H_5$ | $COOCH_3$ |
| $C_3H_7$ | $CH_3$ | $CH_3$ | $C_3H_7$ | $COOCH_3$ |
| $C(CH_3)_3$ | $CH_3$ | $CH_3$ | $C(CH_3)_3$ | $COOC_2H_5$ |
| $OCH_3$ | $CH_3$ | $CH_3$ | $OCH_3$ | $COOCH_3$ |
| $OC_2H_5$ | $CH_3$ | $CH_3$ | $OC_2H_5$ | $COOCH_3$ |
| $CH_3$ | H | H | $CH_3$ | CN |
| $CH_3$ | $C_2H_5$ | $C_2H_5$ | $CH_3$ | CN |
| $CH_3$ | $C_3H_7$ | $C_3H_7$ | $CH_3$ | CN |
| $CH_3$ | $C(CH_3)_3$ | $C(CH_3)_3$ | $CH_3$ | CN |
| $CH_3$ | $OCH_3$ | $OCH_3$ | $CH_3$ | CN |

| $R^1$ | $R^2$ | $R^3$ | $R^4$ | $R^7$ |
|---|---|---|---|---|
| $CH_3$ | $OC_2H_5$ | $OC_2H_5$ | $CH_3$ | CN |
| H | $CH_3$ | $CH_3$ | H | CN |
| $C_2H_5$ | $CH_3$ | $CH_3$ | $C_2H_5$ | CN |
| $C_3H_7$ | $CH_3$ | $CH_3$ | $C_3H_7$ | CN |
| $C(CH_3)_3$ | $CH_3$ | $CH_3$ | $C(CH_3)_3$ | CN |
| $OCH_3$ | $CH_3$ | $CH_3$ | $OCH_3$ | CN |
| $OC_2H_5$ | $CH_3$ | $CH_3$ | $OC_2H_5$ | CN |
| $CH_3$ | H | H | $CH_3$ | CHO |
| $CH_3$ | $C_2H_5$ | $C_2H_5$ | $CH_3$ | CHO |
| $CH_3$ | $C(CH_3)_3$ | $C(CH_3)_3$ | $CH_3$ | CHO |
| $CH_3$ | $OCH_3$ | $OCH_3$ | $CH_3$ | CHO |
| $CH_3$ | $OC_2H_5$ | $OC_2H_5$ | $CH_3$ | CHO |
| H | $CH_3$ | $CH_3$ | H | CHO |
| $C_2H_5$ | $CH_3$ | $CH_3$ | $C_2H_5$ | CHO |
| $C(CH_3)_3$ | $CH_3$ | $CH_3$ | $C(CH_3)_3$ | CHO |
| $OCH_3$ | $CH_3$ | $CH_3$ | $OCH_3$ | CHO |
| $OC_2H_5$ | $CH_3$ | $CH_3$ | $OC_2H_5$ | CHO |
| $CH_3$ | H | H | $CH_3$ | Cl |

| $R^1$ | $R^2$ | $R^3$ | $R^4$ | $R^7$ |
|---|---|---|---|---|
| $CH_3$ | $C_2H_5$ | $C_2H_5$ | $CH_3$ | Cl |
| $CH_3$ | $C(CH_3)_3$ | $C(CH_3)_3$ | $CH_3$ | Cl |
| $CH_3$ | $OCH_3$ | $OCH_3$ | $CH_3$ | Cl |
| $CH_3$ | $OC_2H_5$ | $OC_2H_5$ | $CH_3$ | Br |
| H | $CH_3$ | $CH_3$ | H | Br |
| $C_2H_5$ | $CH_3$ | $CH_3$ | $C_2H_5$ | Br |
| $C(CH_3)_3$ | $CH_3$ | $CH_3$ | $C(CH_3)_3$ | Br |
| $OCH_3$ | $CH_3$ | $CH_3$ | $OCH_3$ | Br |
| $OC_2H_5$ | $CH_3$ | $CH_3$ | $OC_2H_5$ | Br |
| $CH_3$ | H | H | $CH_3$ | COOH |
| $CH_3$ | $C_2H_5$ | $C_2H_5$ | $CH_3$ | COOH |
| $CH_3$ | $C(CH_3)_3$ | $C(CH_3)_3$ | $CH_3$ | COOH |
| $CH_3$ | $OCH_3$ | $OCH_3$ | $CH_3$ | COOH |
| $CH_3$ | $OC_2H_5$ | $OC_2H_5$ | $CH_3$ | COOH |

| $R^1$ | $R^2$ | $R^3$ | $R^4$ | $R^7$ |
|---|---|---|---|---|
| Cl | Cl | Cl | Cl | $NO_2$ |
| $NH_2$ | $NH_2$ | $NH_2$ | $NH_2$ | $SO_3H$ |
| $NHCH_3$ | $NHCH_3$ | $NHCH_3$ | $NHCH_3$ | CN |
| $NH_2$ | Cl | $NH_2$ | Cl | CHO |
| $NH_2$ | Cl | $NH_2$ | Cl | Cl |
| $NH_2$ | Cl | $NH_2$ | Cl | $COCH_3$ |
| $NH_2$ | Cl | $NH_2$ | Cl | COOH |
| $NH_2$ | Cl | $NH_2$ | Cl | $COOCH_3$ |
| $NH_2$ | Cl | $NH_2$ | Cl | $NO_2$ |
| $NH_2$ | Cl | $NH_2$ | Cl | $SO_3H$ |
| $NH_2$ | $NHCH_3$ | $NH_2$ | $NHCH_3$ | CN |
| $NH_2$ | $NHCH_3$ | $NH_2$ | $NHCH_3$ | CHO |
| $NH_2$ | $NHCH_3$ | $NH_2$ | $NHCH_3$ | Cl |
| $NH_2$ | $NHCH_3$ | $NH_2$ | $NHCH_3$ | $COCH_3$ |
| $NH_2$ | $NHCH_3$ | $NH_2$ | $NHCH_3$ | COOH |
| $NH_2$ | $NHCH_3$ | $NH_2$ | $NHCH_3$ | $COOCH_3$ |
| Br | $NHCH_3$ | Br | $NHCH_3$ | $NO_2$ |
| Br | $NHCH_3$ | Br | $NHCH_3$ | $SO_3H$ |

| $R^1$ | $R^2$ | $R^3$ | $R^4$ | $R^7$ |
|---|---|---|---|---|
| Br | $NHCH_3$ | Br | $NHCH_3$ | CN |
| Br | $NHCH_3$ | Br | $NHCH_3$ | CHO |
| Br | $NHCH_3$ | Br | $NHCH_3$ | Br |
| Br | $NHCH_3$ | Br | $NHCH_3$ | $COCH_3$ |
| Br | $NHCH_3$ | Br | $NHCH_3$ | COOH |
| Br | $NHCH_3$ | Br | $NHCH_3$ | $COOCH_3$ |
| $NH_2$ | $CH_3$ | $NH_2$ | $CH_3$ | $NO_2$ |
| $NH_2$ | $CH_3$ | $NH_2$ | $CH_3$ | $SO_3H$ |
| $NH_2$ | $CH_3$ | $NH_2$ | $CH_3$ | CN |
| $NH_2$ | $CH_3$ | $NH_2$ | $CH_3$ | CHO |
| $NH_2$ | $CH_3$ | $NH_2$ | $CH_3$ | Cl |
| $NH_2$ | $CH_3$ | $NH_2$ | $CH_3$ | $COCH_3$ |
| $NH_2$ | $CH_3$ | $NH_2$ | $CH_3$ | COOH |
| $NH_2$ | $CH_3$ | $NH_2$ | $CH_3$ | $COOCH_3$ |
| Cl | $NH_2$ | Cl | $NH_2$ | $NO_2$ |
| Cl | $NH_2$ | Cl | $NH_2$ | $SO_3H$ |
| Cl | $NH_2$ | Cl | $NH_2$ | CN |
| Cl | $NH_2$ | Cl | $NH_2$ | CHO |

The compound of the general formula (III) may be composed in any of a variety of manners, and may be composed, for example, by a series of reaction formula (E), (F) or (G) set forth below:

Reaction Formula (E):

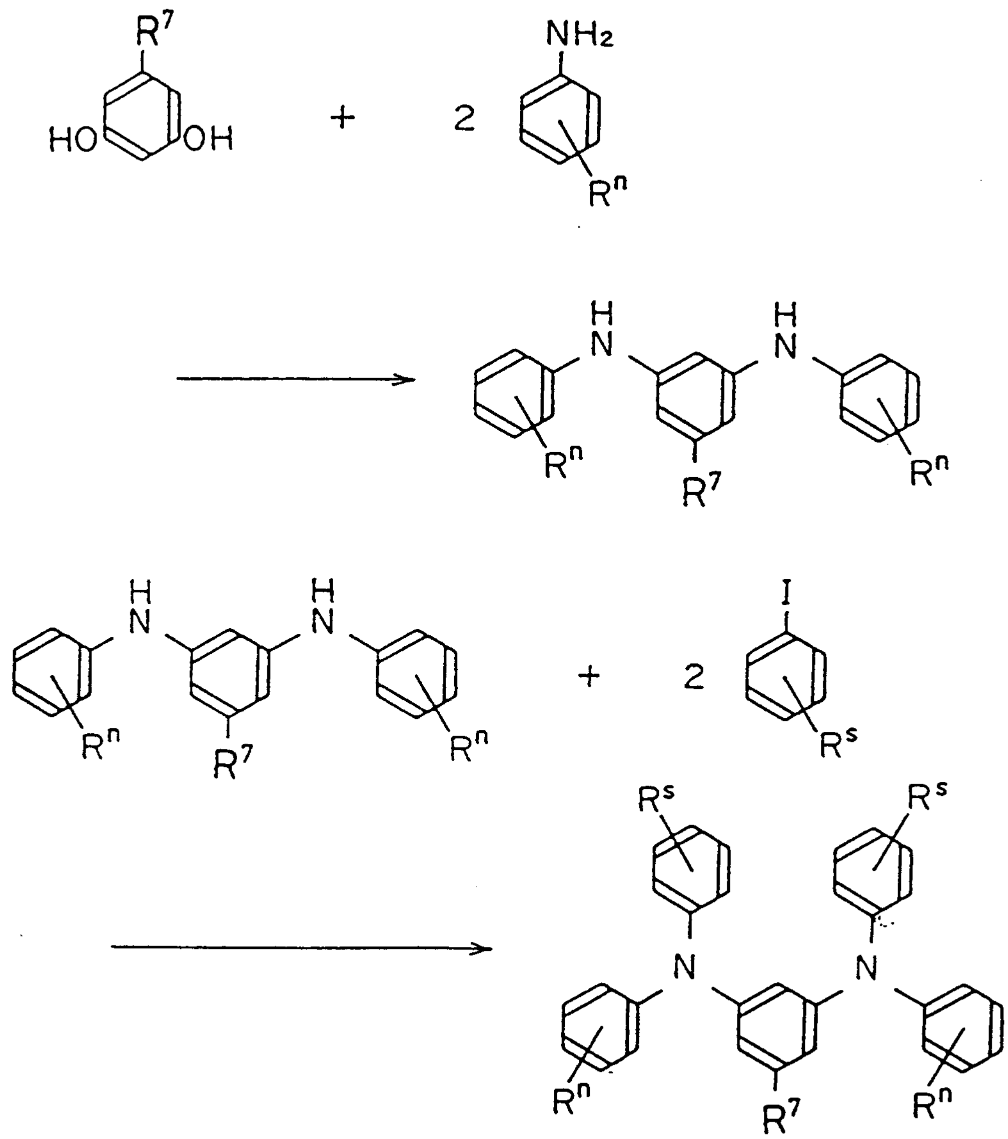

Reaction Formula (F):

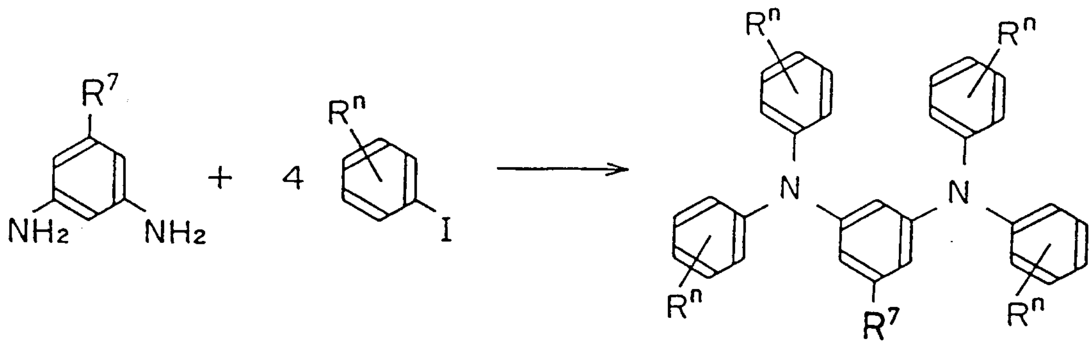

Reaction Formula (G):

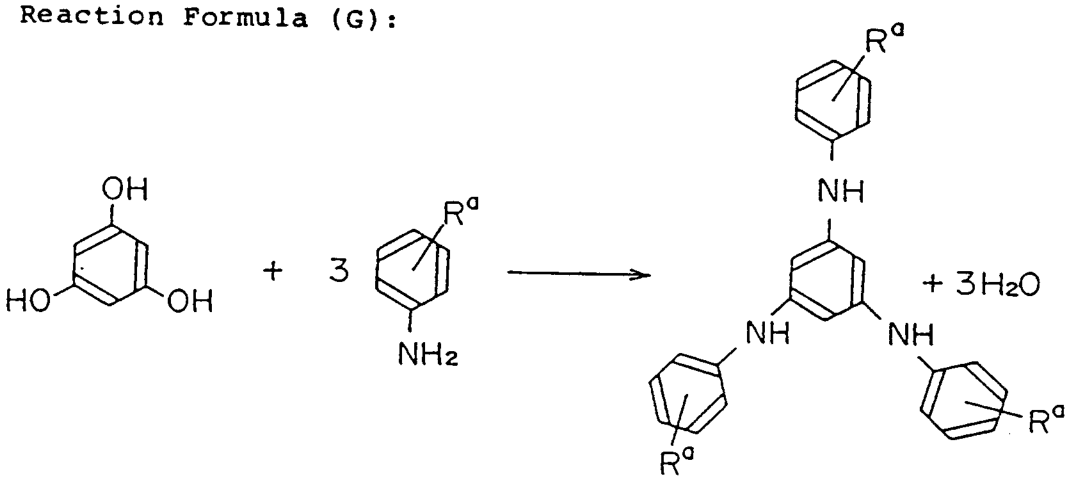

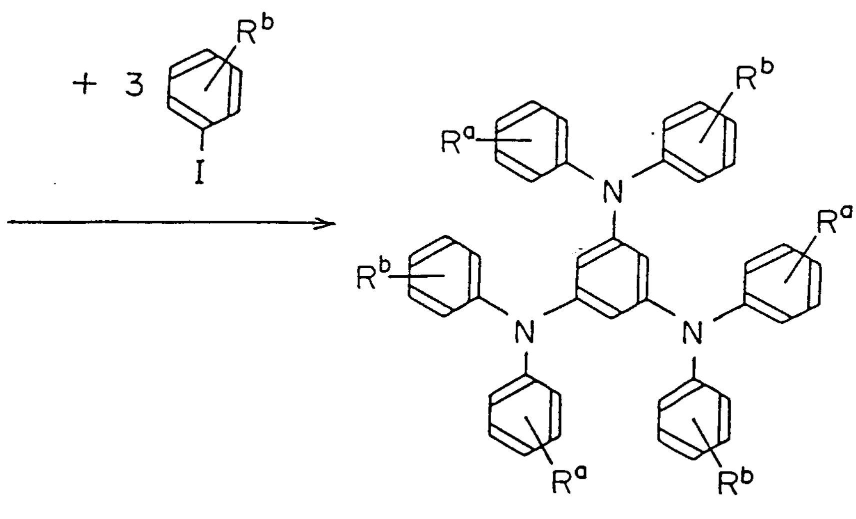

(wherein $R^7$, $R^n$ and $R^s$ are as defined above; $R^a$ and $R^b$ may be same as or different from each other, and each is a hydrogen atom, an alkyl group, an alkoxyl group, a halogen atom, an amino group, an N-substituting amino group, an allyl group or an aryl group).

The reactions of the formulas (E), (F) and (G) may be conducted under the same conditions as those for the reactions of the formulas (A) and (B). Accordingly, the detailed description of the reactions of the

formulas (E), (F) and (G) will be here omitted. The reaction formula (G) represents a compound (III) in which $R^7$ is an N,N-diphenyl substituting amino group.

The compound of each of the general formulas (I), (II) and (III) in accordance with the present invention may be contained, in a binding resin, alone or in combination with other conventional electric charge transferring material, thereby to form a photosensitive layer. As the other electric charge transferring material, there may be used any of conventional electron attractive or donative compounds.

Examples of the electron attractive compound include a diphenoquinone derivative such as 2,6-dimethyl-2',6'di(tert-dibutyl)diphenoquinone or the like, malononitrile, a chiopyran compound, tetracyanoethylene, 2,4,8-trinitrothioxanthene, 3,4,5,7-tetranitro-9-fluorenone, dinitrobenzene, dinitroanthracene, dinitroacridine, nitroanthraquinone, dinitroanthraquinone, succinic anhydride, maleic anhydride, dibromo maleic anhydride and the like.

Examples of the electron donative compound include nitrogen-containing cyclic compounds and condensated polycyclic compounds which include oxadiazole compounds such as 2,5-di(4-methylaminophenyl), 1,3,4-oxadiazole and the like, styryl compounds such as 9-(4-diethylaminostyryl) anthracene and the like, carbazole compounds such as polyvinyl carbazole and the like, pyrazoline compounds such as 1-phenyl-3-(p-dimethylaminophenyl)pyrazoline and the like, hydrazone compounds, triphenylamine compounds, indole compounds, oxazole compounds, isooxazole compounds, thiazole compounds, thiadiazole compounds, imidazole compounds, pyrazole compounds, triazole compounds and the like.

These examples of the electric charge transferring material may be used alone or in combination of plural types. When there is used the electric charge transferring material having film-forming properties such as polyvinyl carbazole or the like, a binding resin is not necessarily required.

The compound of the general formula [I] may be used for a photosensitive layer in an electrophotosensitive material of the so-called single- or multi-layer type.

To form a single-layer type electrophotosensitive material, there may be formed, on a conductive substrate, a photosensitive layer containing the compound of the general formula [I] serving as the electric charge transferring material, an electric charge generating material, a binding resin and the like.

To form a multi-layer type electrophotosensitive material, an electric charge generating layer containing an electric charge generating material is formed on the conductive substrate by vapor deposition, coating or the like, and the electric charge transferring layer containing the compound of the general formula [I] and a binding resin is then formed on the electric charge generating layer. On the contrary, an electric charge transferring layer similar to that above-mentioned may be formed on the conductive substrate, and the electric charge generating layer containing an electric charge generating material may be then formed on the electric charge transferring layer by vapor deposition, coating or the like. Alternately, the electric charge generating layer may be formed by coating a binding resin containing an electric charge generating material and an electric charge transferring material as dispersed therein. According to the present invention, it is a matter of course that the term 'photosensitive layer' contains a photosensitive layer comprising the electric charge generating layer and the electric charge transferring layer.

Examples of the electric charge generating material include selenium, selenium-tellurium, amorphous silicon, pyrylium salt, azo pigment, disazo pigment, anthanthrone pigment, phthalocyanine pigment, indigo pigment, triphenylmethan pigment, surene pigment, toluidine pigment, pyrazoline pigment, perylene pigment, quinacridon pigment, pyrrole pigment and the like. These examples may be used alone or in combination of plural types to present an absorption wavelength in a desired range.

As the binding resin contained in the single- or multi-layer type photosensitive layer, any of a variety of resins may be used. Examples of the binding resin include various polymers which include: thermoplastic resins such as a styrene polymer, a styrene-butadiene copolymer, a styrene-acrylonitrile copolymer, a styrene-maleic acid copolymer, an acrylic polymer, a styrene-acrylic copolymer, polyethylene, an ethylene-vinyl acetate copolymer, chlorinated polyethylene, polyvinyl chloride, polypropylene, a vinyl chloride-vinyl acetate copolymer, polyester, alkyd resin, polyamide, polyurethane, polycarbonate, polyarylate, polysulfon, dialkenyl phthalate resin, ketone resin, polyvinyl butyral resin, polyether resin and the like; crosslinking thermosetting resins such as silicone resin, epoxy resin, phenol resin, urea resin, melamine resin and the like; photosetting resins such as epoxy-acrylate, urethane-acrylate and the like. These polymers may be used alone or in combination of plural types.

A solvent is used when the electric charge generating layer and the electric charge transferring layer are formed with coating means. As such a solvent, there may be used any of a variety of organic solvents. Examples of such organic solvents include: alcohols such as methanol, ethanol, isopropanol, butanol and the like; aliphatic hydrocarbons such as n-hexane, octane, cyclohexane and the like; aromatic hydrocarbons such as benzene, toluene, xylene and the like; halogenated hydrocarbons such as dichloromethane,

dichloroethane, carbon tetrachloride, chlorobenzene and the like; ethers such as dimethyl ether, diethyl ether, tetrahydrofuran, ethylene glycol dimethyl ether, diethylene glycol dimethyl ether and the like; ketones such as acetone, methylethyl ketone, cyclohexanone and the like; esters such as ethyl acetate, methyl acetate and the like; dimethylformaldehyde; dimethylformamide; dimethylsulfoxide and the like. These solvents may be used alone or in combination of plural types.

To improve the electric charge generating layer in sensitivity, there may be used a conventional sensitizer such as tert-phenyl, halonaphtoquinone, acenaphthylene or the like, together with the electric charge generating material. To improve the electric charge transferring and generating materials in dispersibility, dyeability and the like, there may be used a surfactant, a levelling agent and the like.

As the conductive substrate, any of a variety of conductive materials may be used. Examples of such conductive materials include: single metal such as aluminium, copper, tin, platinum, gold, silver, vanadium, molybdenum, chromium, cadmium, titanium, nickel, paradium, indium, stainless copper, brass and the like; plastic material vapor-deposited or laminated with any of the metals above-mentioned; glass material coated with aluminium iodide, tin oxide, indium oxide or the like. The conductive substrate may be made in the form of a sheet or a drum. The substrate itself may be conductive or only the surface of the substrate may be conductive. Preferably, the substrate has a sufficient mechanical strength when used.

The compound of the present invention serving as the electric charge transferring material, and the binding resin may be used at a variety of ratios within such a range as not to prevent the transmission of the electric charge and as to prevent the crystallization of the electric charge transferring material. Preferably, 125 to 200 parts by weight of the compound of the general formula [I] may be used for 100 parts by weight of the binding resin.

The electric charge transferring layer containing the compound of the general formula [I] has a thickness preferably from 2 to 100 $\mu$m and more preferably from 5 to 30 $\mu$m.

When the electric charge generating material is used together with the binding resin, the electric charge generating material and the binding resin may be used in various proportions, but 1 to 150 parts by weight of the binding resin may be preferably used for 10 parts by weight of the electric charge generating material.

The thickness of the electric charge generating layer containing the electric charge generating material above-mentioned is optional, but is preferably from 0.01 to 20$\mu$m and more preferably from 0.1 to 10 $\mu$m. In the single-layer type photosensitive layer containing the compound of the general formula [I] and the electric charge generating material in a single layer, the thickness thereof is optional, but is preferably from 2 to 100 $\mu$m and more preferably from 5 to 30 $\mu$m.

Further, a barrier layer may be formed, in such range as not to injure the characteristics of the photosensitive material, between the substrate and the photosensitive layer in the single-layer type electrophotosensitive material, or between the substrate and the electric charge generating layer, between the substrate and the electric charge transferring layer or between the electric charge generating layer and the electric charge transferring layer in the multi-layer type electrophotosensitive material. Further, a protective layer may be formed on the surface of the photosensitive material.

When the electric charge generating layer and the electric charge transferring layer are formed by a coating method, the electric charge generating material, the binding resin and the like may be prepared as dispersed and mixed with the use of, for example, a roll mill, a ball mill, an atriter, a paint shaker, a supersonic dispenser or the like. Then, the resultant mixture may be applied onto the conductive substrate with the use of any of conventional coating methods, and then allowed to dry. As mentioned earlier, the electric charge generating layer may be formed by vapor-depositing the electric charge generating material.

## Examples

The following description will discuss in detail embodiments of the present invention with reference to examples.

m-Phenylenediamine Compound of General Formula (I)

(1) Synthesis Examples of Electric Charge Transferring Material

**Comparative Example A**

(Synthesis of N,N,N',N'-tetrakis(3-tolyl)-4,6-dichloro-1,3-phenylenediamine)

In the presence of 0.5 g of iodine, 15.7 g of 4,6-dichlororesorcinol and 22.6 g of m-toluidine were reacted at reflux in a stream of nitrogen for three days to give N,N'-bis(3-tolyl)-4,6-dichloro-1,3-phenylenediamine. In the presence of 5.0 g of potassium carbonate and 1.5 g of copper powder, 9.0 g of N,N'-bis(3-tolyl)-4,6-dichloro-1,3-phenylenediamine and 10.9 g of iodotoluene were reacted at reflux in 100 ml of nitrobenzene for 24 hours to prepare N,N,N',N'-tetrakis(3-tolyl)-4,6-dichloro-1,3-phenylenediamine (hereinafter referred to as the Comparative compound No. A). The reaction in the composing process above-mentioned was carried out in accordance with a series of the reaction formula [A] mentioned earlier.

The Comparative compound No. A had a melting point of 110 to 111 °C.

**Synthesis Example 1**

(Synthesis of N,N,N',N'-tetrakis(3-tolyl)-4-amino-1,3-phenylenediamine)

N,N'-bis(3-tolyl)-4-amino-1,3-phenylenediamine was prepared in the same manner as in Comparative Example 1 except for the use of 11.0 g of 4-aminoresorcinol instead of 4,6-dichlororesorcinol used in Example 1. In the presence of 5.0 g of potassium carbonate and 1.5 g of copper powder, 7.6 g of N,N'-bis-(3-tolyl)-4-amino-1,3-phenylenediamine and 10.9 g of iodctoluene were reacted at reflux in 50 ml of nitrobenzene for 24 hours. Thereafter, in the same manner as in Comparative Example 1, there was prepared the compound above-mentioned, N,N,N',N'-tetrakis(3-tolyl)-4-amino-1,3-phenylenediamine (hereinafter referred to as the compound No. 1).

The compound No. 1 had a melting point of 121 to 122 °C.

**Synthesis Example 2**

(Synthesis of N,N,N',N'-tetrakis(3-tolyl)-4-allyl-1,3-phenylenediamine)

N,N'-bis(3-tolyl)-4-allyl-1,3-phenylenediamine was prepared in the same manner as in Comparative Example 1 except for the use of 13.2 g of 4-allylresorcinol instead of 4,6-dichlororesorcinol.

In the presence of 5.0 g of potassium carbonate and 1.5 g of copper powder, 8.2 g of N,N'-bis(3-tolyl)-4-allyl-1,3-phenylenediamine and 10.9 g of iodotoluene were reacted at reflux in 50 ml of nitrobenzene for 24 hours. Thereafter, in the same manner as in Comparative Example 1, there was prepared the compound above-mentioned, N,N,N',N'-tetrakis(3-tolyl)-4-allyl-1,3-phenylenediamine (hereinafter referred to as the compound No. 2).

The compound No. 2 had a melting point of 115 to 116 °C.

**Synthesis Example 3**

(Synthesis of N,N,N',N'-tetrakis(3-tolyl)-4-phenyl-1,3-phenylenediamine)

N,N'-bis(3-tolyl)-4-phenyl-1,3-phenylenediamine was prepared in the same manner as in Comparative Example 1 except for the use of 16.4 g of 4-phenylresorcinol instead of 4,6-dichlororesorcinol. In the presence of 5.0 g of potassium carbonate and 1.5 g of copper powder, 9.1 g of N,N'-bis(3-tolyl)-4-phenyl-1,3-phenylenediamine and 10.9 g of iodotoluene were reacted at reflux in 50 ml of nitrobenzene for 24 hours. Thereafter, in the same manner as in Comparative Example 1, there was prepared the compound above-mentioned, N,N,N',N'-tetrakis(3-tolyl)-4-phenyl-1,3-phenylenediamine (hereinafter referred to as the compound No. 3).

The compound No. 3 had a melting point of 123 to 124 °C.

(2) Preparation of Electrophotosensitive Material

Preparation of Single-Layer Type Electrophotosensitive Material

With the use of a supersonic dispenser, there were mixed and dispersed (i) 8 parts by weight of N,N'-di(3,5-dimethylphenyl)perylene-3,4,9,10-tetracarboxyimido as the electric charge generating material, (ii) 100 parts by weight of any of the compounds of Synthesis Examples 1 to 3 as the electric charge transferring material, (iii) 100 parts by weight of poly-(4,4'-cyclohexylidenediphenyl)carbonate (Polycarbonate Z200 manufactured by Mitsubishi Gas Kagaku Co., Ltd.) as the binding resin and (iv) a predetermined amount of tetrahydrofuran, thereby to prepare a coating solution for a single-layer type photosensitive layer. The coating solution thus prepared was applied to an aluminium roll having an outer diameter 78 mm and a length of 340 mm. The roll was heated and dried in a dark place at 100 °C for 30 minutes. Then, there was prepared a drum-type electrophotosensitive material having a single-layer type photosensitive layer with a thickness of 24 $\mu$m.

Preparation of Multi-Layer Type Electrophotosensitive Material

A ball mill was charged with (i) 100 parts by weight of polyvinyl butyral (Esleck BL1 manufactured by Sekisui Kagaku Kogyo Co., Ltd.) as the binding resin, (ii) 100 parts by weight of oxotitanyl phthalocyanine as the electric charge generating material and (iii) a predetermind amount of tetrahydrofuran. The resultant mixture was agitated and mixed for 24 hours to prepare a coating solution for an electric charge generating layer. The coating solution thus prepared was applied, by an immersion method, to an aluminium drum, which was then dried with hot air at 110 °C for 30 minutes, causing the coating solution to be cured. Thus, an electric charge generating layer with a thickness of 0.5 $\mu$m was formed.

With a homogenizer mixer, there were agitated and mixed (i) 100 parts by weight of a polycarbonate resin (Eupiron manufactured by Mitsubishi Gas Kagaku Kogyo Co., Ltd.), (ii) 100 parts by weight of any of the compounds of Synthesis Examples 1 to 3 as the electric charge transferring material and (iii) a predetermined amount of toluene, thereby to prepare a coating solution for the electric charge transferring layer. The coating solution thus prepared was applied, by an immersion method, to the surface of the electric charge generating layer, which was then dried with hot air at 120 °C for 30 minutes. Thus, there was formed an electric charge transferring layer with a thickness of about 20 $\mu$m, from which a multi-layer type electrophotosensitive material was formed.

Comparative Example B

Single- and multi-layer type electrophotosensitive materials were formed in the same manner as above-mentioned, except for the use of 100 parts by weight of N,N,N',N'-tetrakis(3-tolyl)-1,3-phenylenediamine as the electric charge transferring material.

(3) Evaluation of the Electrophotosensitive Materials

Single-Layer Type Electrophotosensitive Materials

(i) Measurement of Initial Surface Potential $V_{sp}$

Each of the single-layer type electrophotosensitive materials above-mentioned was set in an electrostatic test copier (Gentic Cincia 30M manufactured by Gentic Co.). With the surface of each electrophotosensitive material positively charged, the surface potential $V_{sp}$(V) thereof was measured.

(ii) Measurement of Half-Life Light Exposure and

Residual Potential

Each electrophotosensitive material thus charged was exposed to a halogen lamp (exposure intensity of 0.92 mW/cm$^2$) serving as the exposure light source of aforementioned electrostatic tent copier. The time during which the surface potential $V_{sp}$ is reduced to one half, was then determined, and the half-life light exposure E 1/2 ($\mu$J/cm$^2$) was calculated.

The surface potential of each electrophotosensitive material after the passage of 0.15 second after the light exposure had started, was measured as a residual potential $V_{rp}$(V).

Multi-Layer Type Electrophotosensitive Materials

Each of the multi-layer type electrophotosensitive materials above-mentioned was exposed to monochromatic light (wavelength = 780 nm; exposure intensity = 10 $\mu W/cm^2$; exposure time = 1 second) obtained from light of a Xe lamp disposed in a spectroscope serving as the exposure light source. The surface potential of each electrophotosensitive material after the passage of 0.15 second after the light exposure had started, was measured as residual potential $V_{rp}$. Otherwise, the same measurements were made as those for the single-layer type electrophotosensitive materials above-mentioned.

Tables 1 and 2 show the charging and photosensitive characteristics of the electrophotosensitive materials of Synthesis Examples 1 to 3 and Comparative Examples A and B.

Table 1

| (Single-Layer Type Electrophotosensitive Materials) | | | | |
|---|---|---|---|---|
| Type of Charge Transferring Material (Compound No.) | | $V_{sp}$ (V) | $V_{rp}$ (V) | E 1/2 ($\mu J/cm^2$) |
| Comparative Example A | | | | |
| | Before Exposure<br>After Exposure | 715<br>730 | 136<br>150 | 28.0<br>29.8 |
| No. 1 | Before Exposure<br>After Exposure | 718<br>732 | 135<br>141 | 28.1<br>28.4 |
| No. 2 | Before Exposure<br>After Exposure | 716<br>735 | 139<br>153 | 28.0<br>29.9 |
| No. 3 | Before Exposure<br>After Exposure | 723<br>738 | 130<br>137 | 27.4<br>27.7 |
| Comparative Example B | | | | |
| | Before Exposure<br>After Exposure | 710<br>760 | 140<br>210 | 28.2<br>37.2 |

Table 2

| (Multi-Layer Type Electrophotosensitive Materials) | | | | |
|---|---|---|---|---|
| Type of Charge Transferring Material (Compound No.) | | $V_{sp}$ (V) | $V_{rp}$ (V) | E 1/2 ($\mu J/cm^2$) |
| Comparative Example A | | | | |
| | Before Exposure<br>After Exposure | 713<br>611 | 28<br>47 | 0.60<br>0.66 |
| No. 1 | Before Exposure<br>After Exposure | 726<br>666 | 29<br>30 | 0.60<br>0.60 |
| No. 2 | Before Exposure<br>After Exposure | 730<br>604 | 31<br>43 | 0.61<br>0.63 |
| No. 3 | Before Exposure<br>After Exposure | 718<br>676 | 28<br>32 | 0.59<br>0.60 |
| Comparative Example B | | | | |
| | Before Exposure<br>After Exposure | 710<br>501 | 25<br>62 | 0.61<br>0.71 |

In Tables 1 and 2, the data for 'Before Exposure' represent the initial characteristics before the light is irradiated, while the data for 'After Exposure' represent the characteristics after white light including ultraviolet rays of 400 luxes has been irradiated for 40 minutes.

As shown in Tables 1 and 2, the photosensitive materials using the compound (I) as the electric charge transferring material, are excellent in photostability in that their variations of the initial surface potential $V_{sp}$, half-life light exposure E 1/2 and residual potential $V_{rp}$ before and after light exposre are smaller than those of Comparative Examples.

m-Phenylenediamine Compound of General Formula (II)

(1) Synthesis Examples of Electric Charge Transferring Material

**Synthesis Example 4**

(Synthesis of N,N,N',N'-tetrakis(2-tolyl)-1,3-phenylenediamine)

In the presence of 6.9 g (0.05 mol) of potassium carbonate and 1.5 g of copper power, 2.7 g (0.025 mol) of m-phenylenediamine and 21.8 g (0.1 mol) of o-iodotoluene were reacted at reflux in 100 ml of nitrobenzene for 24 hours to give the compound above-mentioned, N,N,N',N'-tetrakis(2-tolyl)-1,3-phenylenediamine (hereinafter referred to as the compound No. 4).

The following shows the results of elemental analysis of this compound.

| In $C_{34}H_{32}N_2$: | | | |
|---|---|---|---|
| Measured Values -<br>Calculation Values - | H:6.91%,<br>H:6.88%, | C:87.33%,<br>C:87.14%, | N:5.98%<br>N:6.09% |

The compound No. 4 had a melting point of 181 to 183 °C.

**Synthesis Example 5**

(Synthesis of N,N,N',N'-tetrakis(2-methoxyphenyl)-5-methyl-1,3-phenylenediamine)

In the presence of 0.5 g of iodine, 12.4 g of 5-methylresorcinol and 12.0 g of o-methoxy aminobenzene were reacted at reflux in a stream of nitrogen for three days to give N,N'-bis(2-methoxyphenyl)-5-methyl-1,3-phenylenediamine. In the presence of 9.7 g of potassium carbonate and 2 g of copper power, 16.7 g of

N,N'-bis(2-methoxyphenyl)-5-methyl-1,3-phenylenediamine and 11.7 g of o-methoxy iodobenzene were reacted at reflux in 100 ml of nitrobenzene for 24 hours to prepare the compound above-mentioned, N,N,N',N'-tetrakis(2-methoxyphenyl)-5-methyl-1,3-phenylenediamine (hereinafter referred to as the compound No. 5).

The compound No. 5 had a melting point of 126 to 127 ° C.

**Synthesis Example 6**

(Synthesis of N,N,N',N'-tetrakis(2-chlorophenyl)-1,3-phenylenediamine)

In the presence of 6.9 g of potassium carbonate and 1.5 g of copper powder, 2.7 of m-phenylenediamine and 6.0 g of o-chloroiodobenzene were dissolved in 100 ml of nitrobenzene and reacted at reflux in a stream of nitrogen for 24 hours, thereby to prepare the compound above-mentioned, N,N,N',N'-tetrakis(2-chlorophenyl)-1,3-phenylenediamine (hereinafter referred to as the compound No. 6).

The compound No. 6 had a melting point of 122 to 123 ° C.

**Synthesis Example 7**

(Synthesis of N,N,N',N'-tetrakis(2-aminophenyl)-5-methyl-1,3-phenylenediamine)

N,N'-bis(2-aminophenyl)-5-methyl-1,3-phenylenediamine was prepared in the same manner as in Synthesis Example 5 except for the use of 10.8 g of o-phenylenediamine instead of o-methoxyaminobenzene used in Synthesis Example 5. In the presence of 9.7 g of potassium carbonate and 2 g of copper power, 15.2 g of N,N'-bis(2-aminophenyl)-5-methyl-1,3-phenylenediamine and 11.0 g of o-aminoiodobenzene were reacted at reflux in 100 ml of nitrobenzene for 24 hours, thereby to prepare the compound above-mentioned, N,N,N',N'-tetrakis(2-aminophenyl)-5-methyl-1,3-phenylenediamine (hereinafter referred to as the compound No. 7).

The compound No. 7 had a melting point of 144 to 145 ° C.

(2) Preparation and Evaluation of Electrophotosensitive Materials

In the same manner as for the m-phenylenediamine compound (I) above-mentioned, single- and multi-layer type electrophotosensitive materials were prepared with the use of the respective compounds of Synthesis Examples 4 to 7. In the same manner as mentioned earlier, there were measured the initial surface potentials $V_{sp}$, residual potentials $V_{rp}$ and half-life light exposures E 1/2, before and after light exposure, of each of the electrophotosensitive materials thus prepared.

Tables 3 and 4 show the charging and photosensitive characteristics of the electrophotosensitive materials using the compounds of Synthesis Examples 4 to 7, together with those of Comparative Example B.

Table 3

| (Single-Layer Type Electrophotosensitive Materials) | | | | | |
|---|---|---|---|---|---|
| Type of Charge Transferring Material (Compound No.) | | $V_{sp}$ (V) | $V_{rp}$ (V) | E 1/2 ($\mu$J/cm$^2$) |
| No. 4 | Before Exposure<br>After Exposure | 720<br>728 | 135<br>143 | 27.8<br>28.4 |
| No. 5 | Before Exposure<br>After Exposure | 730<br>737 | 138<br>141 | 28.0<br>28.2 |
| No. 6 | Before Exposure<br>After Exposure | 742<br>744 | 140<br>141 | 28.3<br>28.2 |
| No. 7 | Before Exposure<br>After Exposure | 695<br>720 | 143<br>156 | 28.5<br>29.1 |
| Comparative Example B | | | | |
| | Before Exposure<br>After Exposure | 710<br>760 | 140<br>210 | 28.2<br>37.2 |

Table 4

| (Multi-Layer Type Electrophotosensitive Materials) | | | | |
|---|---|---|---|---|
| Type of Charge Transferring Material (Compound No.) | | $V_{sp}$ (V) | $V_{rp}$ (V) | E 1/2 ($\mu$J/cm$^2$) |
| No. 4 | Before Exposure<br>After Exposure | 708<br>640 | 20<br>38 | 0.58<br>0.63 |
| No. 5 | Before Exposure<br>After Exposure | 731<br>677 | 31<br>46 | 0.62<br>0.66 |
| No. 6 | Before Exposure<br>After Exposure | 722<br>680 | 33<br>35 | 0.60<br>0.61 |
| No. 7 | Before Exposure<br>After Exposure | 703<br>619 | 39<br>48 | 0.64<br>0.67 |
| Comparative Example B | | | | |
| | Before Exposure<br>After Exposure | 710<br>501 | 25<br>62 | 0.61<br>0.71 |

m-Phenylenediamine Compound of General Formula (III)

(1) Synthesis Examples of Electric Charge Transferring Material

**Comparative Example A'**

(Synthesis of N,N,N',N'-tetrakis(3-tolyl)-5-methyl-1,3-phenylenediamine)

In the presence of 0.5 g of iodine, 12.5 g (88 millmol) of 5-methylresorcinol (orcin) and 22.6 g of m-toluidine were reacted at reflux in a stream of nitrogen for three days. The reacted product was cooled to a room temperature, and the resultant solid body was washed with 500 ml of methanol to give N,N'-bis(-3-tolyl)-5-methyl-1,3-phenylenediamine. In the presence of 9.7 g of potassium carbonate and 2 g of copper powder, 15.1 g of N,N'-bis(3-tolyl)-5-methyl-1,3-phenylenediamine and 21.8 g of iodotoluene were reacted at reflux in 100 ml of nitrobenzene for 24 hours. Then, nitrobenzene and iodotoluene were removed from the reacted product by distillation of vapor. The residue was washed first with water and then with methanol.

Then, the residue was added to 900 ml of benzene, and the water-soluble matter was collected by filtration and applied to active almina column chromatography using a benzene-hexane mixture (at 1:1) as a developing solvent to obtain the 1st fraction. The 1st fraction was applied to active almina colomn chromatography using a benzene-hexane mixture (at 1:2) as a developing solvent to obtain the 1st fraction. The solvent was removed, and a portion of the residue was dissolved in acetonitrile at an ambient temperature to produce a crystal. The remaining residue was crystallized using the above mentioned crystal as a core, thereby to prepare the compound above-mentioned, N,N,N',N'-trakis(3-toryl)-5-methyl-1,3-phenylenediamine (hereinafter referred to as the Comparative compound A'). The reactions in the synthesis process above-mentioned were carried out in accordance with the reaction formula [E].

The following shows the results of elemental analysis of the compound A'.

| In $C_{35}H_{34}N_2$: | | | |
|---|---|---|---|
| Measured Values - | H:7.13%, | C:87.08%, | N:5.83% |
| Calculation Values - | H:7.10%, | C:87.10%, | N:5.80% |

The compound A' had a melting point of 156 to 157 °C.

**Synthesis Example 8**

(Synthesis of N,N,N',N'-hexakis(4-tolyl)-1,3,5-benzenetriamine)

The compound above-mentioned was composed in accordance with the reaction formula [G] mentioned earlier.

In the presence of 0.5 g of iodine, 12.6 g (0.1 mol) of phloroglucine and 48.2 g (0.45 mol) of p-toluidine were reacted at reflux in a stream of nitrogen for six hours to give N,N',N'-tris(4-tolyl)-1,3,5-benzenetriamine. In the presence of 7.6 g of potassium carbonate and 3 g of copper powder, 14.4 g (0.037 mol) of N,N',N'-tris(4-tolyl)-1,3,5-benzenetriamine and 32 g (147 mol) of p-iodotoluene were reacted at reflux in 150 ml of nitrobenzene for 36 hours, thereby to prepare the compound above-mentioned, N,N,N',N'-hexakis(4-tolyl)-1,3,5-benzenetriamine (hereinafter referred to as the compound No. 8).

The compound No. 8 had a melting point of 235 to 236 °C.

**Synthesis Example 9**

(Synthesis of N,N,N',N'-tetrakis(3-tolyl)-1,3,5-benzenetriamine)

N,N'-bis(3-tolyl)-1,3,5-benzenetriamine was prepared in the same manner as in Comparative Example A' except for the use of 11.0 g of 5-aminoresorcinol instead of 5-methylresorcinol (orcin).

In the presence of 9.7 g of potassium carbonate and 2 g of copper powder, 15.2 g of N,N'-b-is(3-tolyl)-1,3,5-benzenetriamine and 21.8 g of iodotoluene were reacted at reflux in 100 ml of nitrobenzene for 24 hours. Thereafter, in the same manner as in Comparative Example A', there was prepared the compound above-mentioned, N,N,N',N'-tetrakis(3-tolyl)-1,3,5-benzenetriamine (hereinafter referred to as the compound No. 9).

The compound No. 9 had a melting point of 186 to 187 °C.

**Synthesis Example 10**

(Synthesis of N,N,N',N'-tetrakis(3-tolyl)-5-allyl-1,3-phenylenediamine)

N,N'-bis(3-tolyl)-5-allyl-1,3-phenylenediamine was prepared in the same manner as in Comparative Example A' except for the use of 13.2 g of 5-allylresorcinol instead of 5-methylresorcinol (orcin). In the presence of 9.7 g of potassium carbonate and 2 g of copper powder, 16.4 g of N,N'-bis(3-tolyl)-5-allyl-1,3-phenylenediamine and 21.8 g of iodotoluene were reacted at reflux in 100 ml of nitrobenzene for 24 hours. Thereafter, in the same manner as in Comparative Example A', there was prepared the compound above-mentioned, N,N,N',N'-tetrakis(3-tolyl)-5-allyl-1,3-phenylenediamine (hereinafter referred to as the compound No. 10).

The compound No. 10 had a melting point of 128 to 129 °C.

**Synthesis Example 11**

(Synthesis of N,N,N',N'-tetrakis(3-tolyl)-5-phenyl-1,3-phenylenediamine)

N,N'-bis(3-tolyl)-5-phenyl-1,3-phenylenediamine was prepared in the same manner as in Comparative Example 11 except for the use of 16.4 g of 5-phenylresorcinol instead of 5-methylresorcinol (orcin). In the presence of 9.7 g of potassium carbonate and 2 g of copper powder, 18.2 g of N,N'-bis(3-tolyl)-5-phenyl-1,3-phenylenediamine and 21.8 g of iodotoluene were reacted at reflux in 100 ml of nitrobenzene for 24 hours. Thereafter, in the same manner as in Comparative Example A', there was prepared the compound above-mentioned, N,N,N',N'-tetrakis(3-tolyl)-5-phenyl-1,3-phenylenediamine (hereinafter referred to as the compound No. 11).

The compound No. 11 had a melting point of 176 to 177 °C.

**Synthesis Example 12**

(Synthesis of N,N,N',N'-tetrakis(3-tolyl)-5-nitro-1,3-phenylenediamine)

In the presence of 0.5 g of iodine, 13.6 g of 5-nitroresorcinol(5-nitro-1,3-benzenediol), 22.6 of m-toluidine were reacted at reflux in a stream of nitrogen for three days. The reacted product was cooled to a room temperature, and the resultant solid body was washed with 500 ml of methanol to give N,N'-bis(3-tolyl)-5-nitro-1,3-phenylenediamine. Then, 16.7 g of N,N'-bis(3-tolyl)-5-nitro-1,3-phenylenediamine, 21.8 g of iodotoluene, 9.7 g of potassium carbonate and 2 g of copper powder were reacted at reflux in 100 ml of nitrobenzene for 24 hours. Then, nitrobenzene and iodotoluene were removed from the reacted product by distillation of vapor. The residue was washed first with water and then with methanol. Then, the residue was added to 900 ml of benzene, and the water-soluble matter was collected by filtration and applied to active almina column chromatography using a benzene-hexane mixture at 1:1 as a developing solvent to obtain the 1st fraction. The 1st fraction was applied to active almina colomn chromatography using a benzene-hexane mixture at 1:2 as a developing solvent to obtain the 1st fraction. The solvent was removed and a portion of the residue was dissolved in acetonitrile at an ambient temperature to produce a crystal. The remaining residue was crystallized using the above-mentioned crystal as a core, thereby to prepare the compound above-mentioned, N,N,N',N'-trakis(3-toryl)-5-nitro-1,3-phenylenediamine (hereinafter referred to as the compound No. 12).

The compound No. 12 had a melting point of 119 to 120 °C.

**Synthesis Example 13**

(Synthesis of N,N,N',N'-tetrakis(3-tolyl)-5-sulfo-1,3-phenylenediamine)

N,N'-bis(3-tolyl)-5-sulfo-1,3-phenylenediamine was prepared in the same manner as in Synthesis Example 12 except for the use of 16.8 g of 5-sulforesorcinol(3,5-dihydroxybenzenesulfonic acid) instead of 5-nitroresorcinol. Then, 9.3 g of N,N'-bis(3-tolyl)-5-sulfo-1,3-phenylenediamine, 10.9 g of iodotoluene, 5 g of potassium carbonate and 1.2 g of copper powder were reacted at reflux in 50 ml of nitrobenzene for 24 hours. Thereafter, in the same manner as in Synthesis Example 12, there was prepared the compound above-mentioned, N,N,N',N'-tetrakis(3-tolyl)-5-sulfo-1,3-phenylenediamine (hereinafter referred to as the compound No. 13).

The compound No. 13 had a melting point of 133 to 134 °C.

**Synthesis Example 14**

(Synthesis of N,N,N',N'-tetrakis(3-tolyl)-5-cyano-1,3-phenylenediamine)

N,N'-bis(3-tolyl)-5-cyano-1,3-phenylenediamine was prepared in the same manner as in Synthesis Example 12 except for the use of 11.9 g of 5-cyanoresorcinol(3,5-dihydroxybenzonitrile) instead of 5-nitroresorcinol. Then, 7.8 g of N,N'-bis(3-tolyl)-5-cyano-1,3-phenylenediamine, 10.9 g of iodotoluene, 5 g of potassium carbonate and 1.2 g of copper powder were reacted at reflux in 50 ml of nitrobenzene for 24 hours. Thereafter, in the same manner as in Synthesis Example 12, there was prepared the compound above-mentioned, N,N,N',N'-tetrakis(3-tolyl)-5-cyano-1,3-phenylenediamine (hereinafter referred to as the compound No. 14).

The compound No. 14 had a melting point of 118 to 119 °C.

**Synthesis Example 15**

(Synthesis of N,N,N',N'-tetrakis(3-tolyl)-5-formyl-1,3-phenylenediamine)

N,N'-bis(3-tolyl)-5-formyl-1,3-phenylenediamine was prepared in the same manner as in Synthesis Example 12 except for the use of 12.1 g of 5-formylresorcinol(3,5-dihydroxybenzaldehyde) instead of 5-nitroresorcinol.

Then, 7.9 g of N,N'-bis(3-tolyl)-5-formyl-1,3-phenylenediamine, 10.9 g of iodotoluene, 5 g of potassium carbonate and 1.2 g of copper powder were reacted at reflux in 50 ml of nitrobenzene for 24 hours. Thereafter, in the same manner as in Synthesis Example 12, there was prepared the compound above-mentioned, N,N,N',N'-tetrakis(3-tolyl)-5-formyl-1,3-phenylenediamine (hereinafter referred to as the compound No. 15).

The compound No. 15 had a melting point of 120 to 121 °C.

**Synthesis Example 16**

(Synthesis of N,N,N',N'-tetrakis(3-tolyl)-5-acetyl-1,3-phenylenediamine)

N,N'-bis(3-tolyl)-5-acetyl-1,3-phenylenediamine was prepared in the same manner as in Synthesis Example 12 except for the use of 13.4 g of 5-acetylresorcinol(3,5-dihydroxyacetophenone) instead of 5-nitroresorcinol. Then, 8.3 g of N,N'-bis(3-tolyl)-5-acetyl-1,3-phenylenediamine, 10.9 g of iodotoluene, 5 g of potassium carbonate and 1.2 g of copper powder were reacted at reflux in 50 ml of nitrobenzene for 24 hours. Thereafter, in the same manner as in Synthesis Example 12, there was prepared the compound above-mentioned, N,N,N',N'-tetrakis(3-tolyl)-5-acetyl-1,3-phenylenediamine (hereinafter referred to as the compound No. 16).

The compound No. 16 had a melting point of 122 to 123 °C.

**Synthesis Example 17**

(Synthesis of N,N,N',N'-tetrakis(3-tolyl)-5-ethoxycarbonyl-1,3-phenylenediamine)

N,N'-bis(3-tolyl)-5-ethoxycarbonyl-1,3-phenylenediamine was prepared in the same manner as in Synthesis Example 12 except for the use of 16.0 g of 3,5-dihydroxy-ethyl benzoate) instead of 5-nitroresorcinol. Then, 9.0 g of N,N'-bis(3-tolyl)-5-ethoxycarbonyl-1,3-phenylenediamine, 10.9 g of iodotoluene, 5 g of potassium carbonate and 1.2 g of copper powder were reacted at reflux in 50 ml of nitrobenzene for 24 hours. Thereafter, in the same manner as in Synthesis Example 12, there was prepared the compound above-mentioned, N,N,N',N'-tetrakis(3-tolyl)-5-ethoxycarbonyl-1,3-phenylenediamine (hereinafter referred to as the compound No. 17).

The compound No. 17 had a melting point of 136 to 137 °C.

(2) Preparation and Evaluation of Electrophotosensitive Materials

In the same manner as for the m-phenylenediamine compound (I) mentioned earlier, single- and multi-layer type electrophotosensitive materials were prepared with the use of the respective compounds of Synthesis Examples 8 to 17. In the same manner as mentioned earlier, there were measured the initial surface potentials $V_{sp}$, residual potentials $V_{rp}$ and half-life light exposures E 1/2, before and after light exposure, of each of the electrophotosensitive materials thus prepared.

Tables 5 and 6 show the measured data together with those of Comparative Examples A' and B.

As shown in Tables 5 and 6, the photosensitive materials using the compound of electric charge transferring material in accordance with the present invention, are excellent in photostability in that their variations of the initial surface potential $V_{sp}$, half-life light exposure E 1/2 and residual potential $V_{rp}$ before and after light exposure are smaller than those of Comparative Examples.

Table 5 (1/2)

(Single-Layer Type Electrophotosensitive Materials)

| Type of Charge Transferring Material (Compound No.) | | $V_{sp}$ (V) | $V_{rp}$ (V) | E 1/2 (μJ/cm$^2$) |
|---|---|---|---|---|
| Comparative Example A' | Before Exposure | 715 | 136 | 28.0 |
| | After Exposure | 730 | 150 | 29.8 |
| No. 8 | Before Exposure | 730 | 120 | 26.3 |
| | After Exposure | 739 | 128 | 27.2 |
| No. 9 | Before Exposure | 712 | 136 | 28.1 |
| | After Exposure | 739 | 153 | 28.9 |
| No. 10 | Before Exposure | 701 | 129 | 27.1 |
| | After Exposure | 737 | 155 | 29.1 |
| No. 11 | Before Exposure | 705 | 130 | 27.3 |
| | After Exposure | 711 | 135 | 27.4 |
| No. 12 | Before Exposure | 728 | 136 | 27.7 |
| | After Exposure | 733 | 139 | 27.9 |
| No. 13 | Before Exposure | 711 | 142 | 28.3 |
| | After Exposure | 715 | 147 | 28.5 |
| No. 14 | Before Exposure | 731 | 127 | 27.3 |
| | After Exposure | 730 | 129 | 27.2 |

Table 5 (2/2)

(Single-Layer Type Electrophotosensitive Materials)

| Type of Charge Transferring Material (Compound No.) | | $V_{sp}$ (V) | $V_{rp}$ (V) | E 1/2 ($\mu J/cm^2$) |
|---|---|---|---|---|
| No. 15 | Before Exposure | 704 | 140 | 28.0 |
| | After Exposure | 737 | 152 | 28.9 |
| No. 16 | Before Exposure | 720 | 131 | 27.5 |
| | After Exposure | 732 | 134 | 27.6 |
| No. 17 | Before Exposure | 735 | 136 | 28.0 |
| | After Exposure | 733 | 137 | 28.1 |
| Comparative Example B | | | | |
| | Before Exposure | 710 | 140 | 28.2 |
| | After Exposure | 760 | 210 | 37.2 |

Table 6 (1/2)

(Multi-Layer Type Electrophotosensitive Materials)

| Type of Charge Transferring Material (Compound No.) | | $V_{sp}$ (V) | $V_{rp}$ (V) | E 1/2 (μJ/cm$^2$) |
|---|---|---|---|---|
| | Before Exposure | 713 | 28 | 0.60 |
| | After Exposure | 611 | 47 | 0.66 |
| Comparative example A' | | | | |
| No. 8 | Before Exposure | 724 | 25 | 0.57 |
| | After Exposure | 655 | 36 | 0.60 |
| No. 9 | Before Exposure | 721 | 33 | 0.60 |
| | After Exposure | 603 | 36 | 0.62 |
| No. 10 | Before Exposure | 735 | 34 | 0.61 |
| | After Exposure | 757 | 49 | 0.67 |
| No. 11 | Before Exposure | 733 | 29 | 0.59 |
| | After Exposure | 683 | 33 | 0.62 |
| No. 12 | Before Exposure | 732 | 33 | 0.63 |
| | After Exposure | 669 | 41 | 0.64 |
| No. 13 | Before Exposure | 737 | 39 | 0.62 |
| | After Exposure | 639 | 45 | 0.65 |
| No. 14 | Before Exposure | 713 | 28 | 0.58 |
| | After Exposure | 676 | 32 | 0.60 |

Table 6 (2/2)

(Multi-Layer Type Electrophotosensitive Materials)

| Type of Charge Transferring Material (Compound No.) | | $V_{sp}$ (V) | $V_{rp}$ (V) | E 1/2 (μJ/cm²) |
|---|---|---|---|---|
| No. 15 | Before Exposure | 730 | 44 | 0.63 |
| | After Exposure | 621 | 47 | 0.64 |
| No. 16 | Before Exposure | 722 | 36 | 0.64 |
| | After Exposure | 680 | 42 | 0.64 |
| No. 17 | Before Exposure | 736 | 29 | 0.58 |
| | After Exposure | 681 | 32 | 0.60 |
| Comparative Example B | | | | |
| | Before Exposure | 710 | 25 | 0.61 |
| | After Exposure | 501 | 62 | 0.71 |

## Claims

**1.** A m-phenylenediamine compound represented by the following general formula (I):

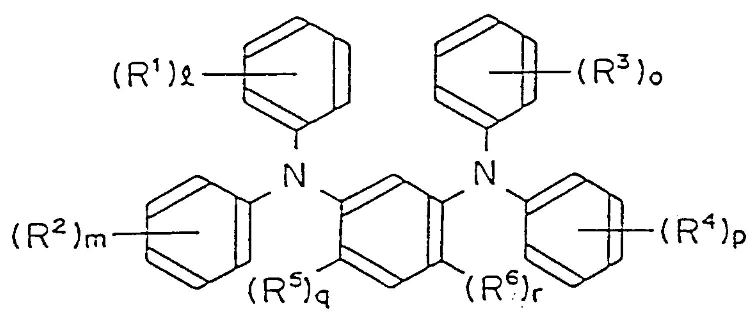

(I)

wherein $R^1$, $R^2$, $R^3$ and $R^4$ are the same as or different from one another, and each is an alkyl group, an alkoxyl group, a halogen atom, an amino group or an N-substituting amino group; l, m, o and p are the same as or different from one another, and each is an integer from 0 to 5; $R^5$ and $R^6$ are the same as or different from each other and each is an amino group, an N-substituting amino group, an alkenyl group or an aryl group; each of q and r is 0 or 1, but q and r should not be 0 simultaneously.

2. A m-phenylenediamine compound represented by the following general formula (II):

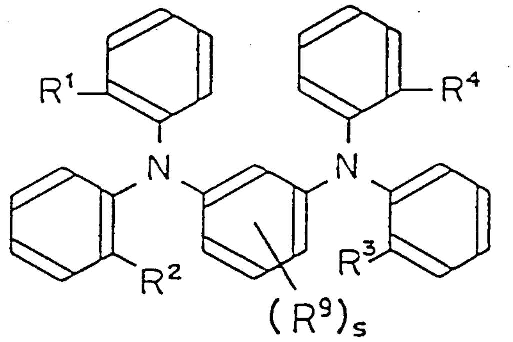

(II)

wherein $R^1$, $R^2$, $R^3$ and $R^4$ are the same as or different from one another and each is an alkyl group, an alkoxyl group, a halogen atom, an amino group or an N-substituting amino group; $R^9$ is an alkyl group, an alkoxyl group, a halogen atom, an amino group, an N-substituting amino group, an alkenyl group or an aryl group; s is an integer from 0 to 4.

3. A m-phenylenediamine compound represented by the following general formula (III):

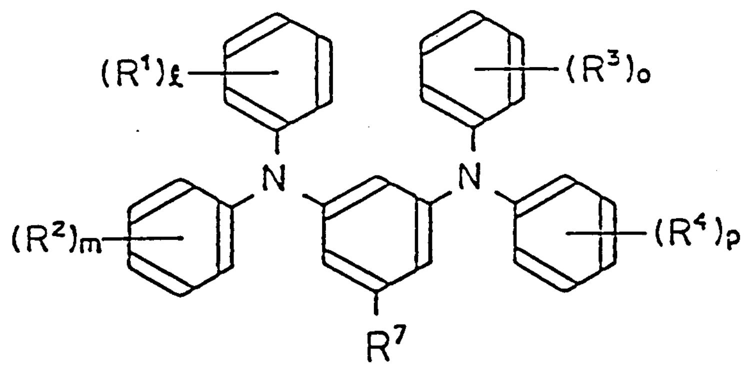

(III)

wherein $R^1$, $R^2$, $R^3$ and $R^4$ are the same as or different from one another and each is an alkyl group, an alkoxyl group, a halogen atom, an amino group or an N-substituting amino group; l, m, o and p are the same as or different from one another and each is an integer from 0 to 5; $R^7$ is an amino group, an alkenyl group, an aryl group or an electron attractive group, provided that the electron attractive group does not include a carboxyl group.

4. A m-phenylenediamine compound according to Claim 3, wherein the electron attractive group is a group selected from the group consisting of nitro, sulfo, cyano, $-COR^8$ (therein $R^8$ is a hydrogen atom, an alkyl group or an amino group) and esterified carboxyl groups.

5. An electrophotosensitive material containing a conductive substrate having thereon a photosensitive layer which contains the m-phenylenediamine compound according to Claim 1, 2 or 3.

**Patentansprüche**

1. m-Phenylendiaminverbindung, die durch die nachstehende allgemeine Formel (I) dargestellt ist:

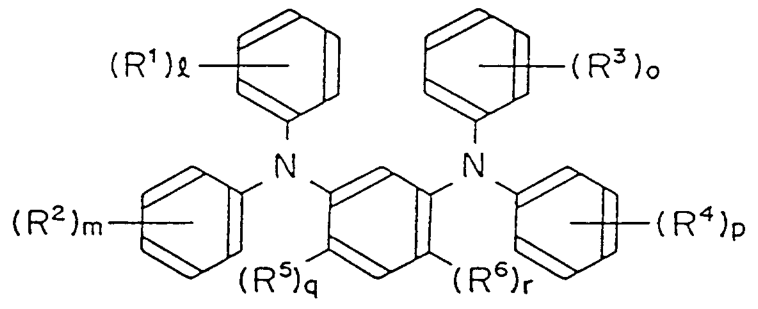

(I)

wobei $R^1$, $R^2$, $R^3$ und $R^4$ jeweils gleich oder voneinander verschieden sind und jedes eine Alkylgruppe, eine Alkoxylgruppe, ein Halogenatom, eine Aminogruppe oder eine N-substituierende Aminogruppe ist; l, m, o und p jeweils gleich oder voneinander verschieden sind und jedes eine ganze Zahl von 0 bis 5 ist; $R^5$ und $R^6$ jeweils gleich oder voneinander verschieden sind und jedes eine Aminogruppe, eine N-substituierende Aminogruppe, eine Alkenylgruppe oder eine Arylgruppe ist; q und r jeweils 0 oder 1 sind, wobei aber q und r nicht gleichzeitig 0 sein sollten.

2. m-Phenylendiaminverbindung, die durch die nachstehende allgemeine Formel (II) dargestellt ist:

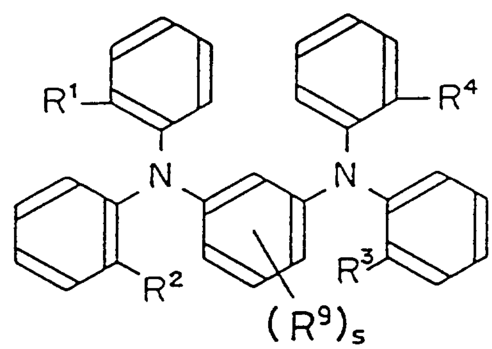

(II)

wobei $R^1$, $R^2$, $R^3$ und $R^4$ jeweils gleich oder voneinander verschieden sind und jedes eine Alkylgruppe, eine Alkoxylgruppe, ein Halogenatom, eine Aminogruppe oder eine N-substituierende Aminogruppe ist; $R^9$ eine Alkylgruppe, eine Alkoxylgruppe, ein Halogenatom, eine Aminogruppe, eine N-substituierende Aminogruppe, eine Alkenylgruppe oder eine Arylgruppe ist; s eine ganze Zahl von 0 bis 4 ist.

3. m-Phenylendiaminverbindung, die durch die nachstehende allgemeine Formel (III) dargestellt ist:

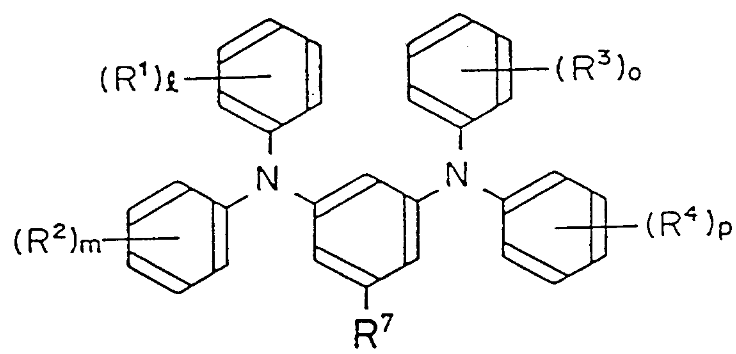

(III)

wobei $R^1$, $R^2$, $R^3$ und $R^4$ jeweils gleich oder voneinander verschieden sind und jedes eine Alkylgruppe,

eine Alkoxylgruppe, ein Halogenatom, eine Aminogruppe oder eine N-substituierende Aminogruppe ist; l, m, o und p gleich oder voneinander verschieden sind und jedes eine ganze Zahl von 0 bis 5 ist; $R^7$ eine Aminogruppe, eine Alkenylgruppe, eine Arylgruppe oder eine elektronenanziehende Gruppe ist mit der Maßgabe, daß die elektronenanziehende Gruppe keine Carboxylgruppe aufweist.

**4.** m-Phenylendiaminverbindung nach Anspruch 3, wobei die elektronenanziehende Gruppe eine Gruppe ist, die aus der Gruppe ausgewählt ist, die aus Nitro-, Sulfo-, Cyano-, $-COR^8$ (wobei $R^8$ ein Wasserstoffatom, eine Alkylgruppe oder eine Aminogruppe ist) und veresterten Carboxylgruppen besteht.

**5.** Elektrophotosensitives Material, das ein leitfähiges Substrat aufweist, das eine photosensitive Schicht trägt, die die m-Phenylendiaminverbindung nach Anspruch 1, 2 oder 3 enthält.

**Revendications**

**1.** Composé m-phénylènediamine représenté par la formule générale (I) ci-après :

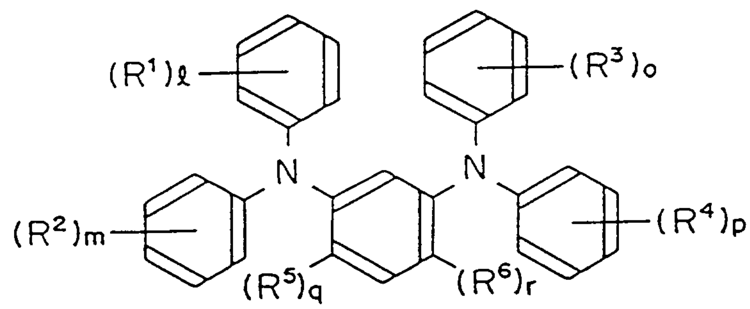

(I)

dans laquelle $R^1$, $R^2$, $R^3$ et $R^4$, qui peuvent être identiques ou différents les uns des autres, représentent chacun un groupe alkyle, un groupe alcoxy, un atome d'halogène, un groupe amino ou un groupe amino N-substitué ; l, m, o et p, qui peuvent être identiques ou différents les uns des autres, représentent chacun un nombre entier compris entre 0 et 5 ; $R^5$ et $R^6$ , qui peuvent être identiques ou différents l'un de l'autre, représentent chacun un groupe amino, un groupe amino N-substitué, un groupe alcényle ou un groupe aryle ; chacun de q et r vaut 0 ou 1, mais q et r ne peuvent être simultanément égaux à 0.

**2.** Composé m-phénylènediamine représenté par la formule générale (II) ci-après :

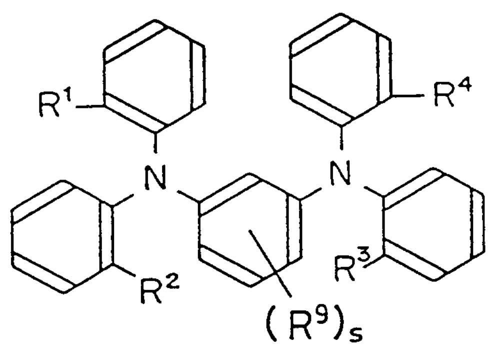

(II)

dans laquelle $R^1$, $R^2$, $R^3$ et $R^4$, qui peuvent être identiques ou différents les uns des autres, représentent chacun un groupe alkyle, un groupe alcoxy, un atome d'halogène, un groupe amino ou un groupe amino N-substitué ; $R^9$ est un groupe alkyle, un groupe alcoxy, un atome d'halogène, un groupe amino, un groupe amino N-substitué, un groupe alcényle ou un groupe aryle ; s est un nombre entier compris entre 0 et 4.

**3.** Composé m-phénylènediamine représenté par la formule générale (III) ci-après :

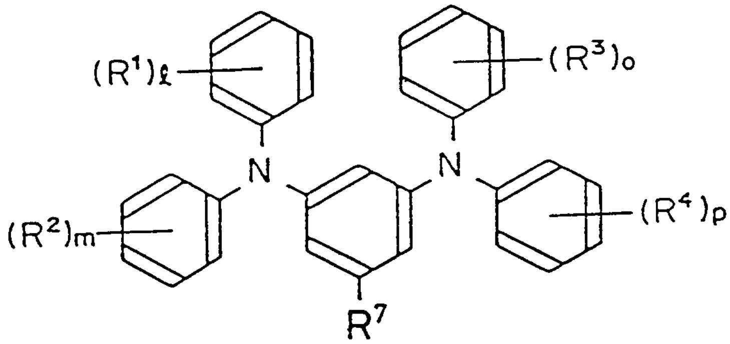

(III)

dans laquelle $R^1$, $R^2$, $R^3$ et $R^4$, qui peuvent être identiques ou différents les uns des autres, représentent chacun un groupe alkyle, un groupe alcoxy, un atome d'halogène, un groupe amino ou un groupe amino N-substitué ; l, m, o et p, qui peuvent être identiques ou différents les uns des autres, représentent chacun un nombre entier compris entre 0 et 5 ; $R^7$ est un groupe amino, un groupe alcényle, un groupe aryle ou un groupe capteur d'électrons, du moment que le groupe capteur d'électrons ne contient pas de fragment carboxyle.

**4.** Composé m-phénylènediamine selon la revendication 3, dans lequel le groupe capteur d'électrons est choisi dans l'ensemble constitué par les groupes nitro, sulfo, cyano, $-COR^8$ (où $R^8$ est un atome d'hydrogène, un groupe alkyle ou un groupe amino), et les groupes carboxyle estérifiés.

**5.** Matériau électrophotosensible contenant un substrat conducteur sur lequel on a déposé une couche photosensible qui contient le composé m-phénylènediamine selon la revendication 1, 2 ou 3.